# EUROPEAN PATENT SPECIFICATION

(11) **EP 4 068 960 B1**
(45) Date of publication and mention of the grant of the patent: **11.03.2026**
(21) Application number: 20812283.8
(22) Date of filing: 24.11.2020
(51) Int. Cl.: B65D 81/38, F17C 13/06, A01N 1/145

(54) **BUNG FOR INSULATING A CONTAINER AND COOLING METHODS**
STOPFEN ZUM ISOLIEREN EINES BEHÄLTERS UND KÜHLVERFAHREN
BOUCHON POUR ISOLER UN RÉCIPIENT ET PROCÉDÉS DE REFROIDISSEMENT

(30) Priority: 03.12.2019 GB 201917626
(43) Date of publication of application: 12.10.2022
(73) Proprietor: Biosafe SA, 1274 Grens (CH)
(72) Inventor: LAMB, Stephen, Cambridge CB24 9BZ (GB); CREASEY, Christopher, Cambridge CB24 9BZ (GB); MOLONY, David, Cambridge CB24 9BZ (GB); MORRIS, George, Cambridge CB24 9BZ (GB); KILBRIDE, Peter, Cambridge CB24 9BZ (GB); MENEGHEL, Julie, Cambridge CB24 9BZ (GB)
(74) Representative: Bedford, Grant Richard
(86) International application number: PCT/EP2020/083216
(87) International publication number: WO 2021/110480

(56) References cited:
- CN-U- 209 136 313
- DE-A1- 2 553 939
- DE-C- 816 592
- US-A1- 2019 063 688

## Description

### FIELD

The present disclosure relates to a bung device for insulating the interior of a container apparatus from the ambient environment, and methods of cooling such a container.

### BACKGROUND

New cell and gene therapies are successfully treating different cancers. There is a global demand for these therapies. Often, a patient is located in a different country to the cell production site, meaning that biological material such as tissue and cells are often cryogenically frozen (for example at a temperature of less than -120°C) so that they can be stored and shipped. Cryogenic freezing is used because the cells do not survive long enough to be suitable for treatment when they are chilled. The biological material must be maintained at cryogenic temperatures during transportation.

It is therefore necessary to transport cell samples at cryogenic temperatures. Existing methods of transporting cell samples at cryogenic temperatures use a "dry shipper". A dry shipper is a vacuum-insulated storage vessel (or "Dewar") which contains a zeolite material. The zeolite material absorbs liquid nitrogen (at -196°C) which provides a cold source for the cell samples being transported in the dry shipper. Absorption of the liquid nitrogen by the zeolite prevents the liquid nitrogen from being spilt or splashed from the vessel. The dry shipper maintains a steady temperature of -196°C for many days as the nitrogen constantly evaporates from the zeolite. A dry shipper typically provides 4 to 10 days of cryogenic stand-by time before warming up to ambient temperature.

Existing dry shippers present health and safety challenges to the couriers, airlines and clinics that handle them. For example, the constant evaporation of liquid nitrogen from the zeolite presents a risk of suffocating users as the evaporated nitrogen displaces oxygen from the ambient environment. The risk of asphyxiation means that the dry shipper must be carefully stored and transported.

Biological samples must remain free from contamination during transportation. However, there is a risk that the biological sample can be contaminated from bacteria, viruses, fungi and other microbes, as well as DNA, RNA and cell fragments from biological samples that have previously been transported in the same shipping device. The biological sample may also be contaminated by contamination of the cooling medium (e.g. dry ice or liquid nitrogen), or by contamination from the ambient environment. To minimise the risks of contamination, existing dry shippers are often warmed up to ambient temperature and cleaned between uses. Cleaning can involve covering the surfaces of a dry shipper with a liquid cleaning product such as water, ethanol, methanol or a detergent, or a gaseous cleaning reagent such as hydrogen peroxide, or a combination of these methods.

Various known containers are described, for example, in DE2553939A1 which discloses a bung according to the preamble of claim 1 , CN 209 136 313 U, US 2019/063688 A1 and DE 816 592 C.

A shipping container for cryopreserved biological samples is also described in WO 2018/115833. One implementation of the shipping container described in WO 2018/115833 includes a thermal mass which is shaped to at least partly contain or hold or surround one or more cryopreserved samples. The thermal mass is used to slow down the rate of temperature change (increase) within the cavity of the shipping container. A heat exchanger is located within the cavity of the shipping container. The heat exchanger is attached to a Stirling cryocooler located on the lid of the shipping container. The Stirling cryocooler is used to remove heat from the cavity. The shipping container comprises a gravitational thermal diode which is operable in a first state to provide cooling to the cavity and in a second state to impair heat transfer into the cavity. The use of a gravitational thermal diode means that the diode (and therefore the shipping container) is required to be maintained in an upright position in order to maintain a temperature gradient between its uppermost and lowermost extremities. For this reason, the lid of the shipping container may be equipped with a tilt sensor to ensure that the shipping container is maintained in an upright position.

During transport, however, it is not uncommon for shipping containers to fall onto their sides or to be transported in an upside-down position. This reduces the effectiveness of the thermal diode used in the shipping container described in WO 2018/115833, meaning that there is a risk that cryogenic temperatures are not maintained during transport, leading to loss of the biological sample. For example, when the shipping container is on its side, the cold source (which was previously located close to the base of the container) shifts so that it is located along a side wall of the shipping container (i.e. closer to the ambient region at the top of the shipping container). This reduces the cooling effect provided by the cold source. In addition, given that the Stirling cryocooler is part of the shipping container described in WO 2018/115833, the Stirling cryocooler is also transported when the biological sample is shipped. Shipping the Stirling cryocooler leads to increased costs. In addition, some clinics may not recognise the requirement to switch on the Stirling cryocooler in order to maintain the cryogenic temperatures within the shipping container. If the Stirling cryocooler is not switched on, then the container may heat up to ambient temperature before the biological sample has been used, leading to loss of the sample.

As explained above, the thermal mass of the shipping container described in WO 2018/115833 is used to slow down the rate of temperature increase within the cavity of the shipping container. The thermal mass takes a long time to be cooled down from ambient temperature to cryogenic temperatures in light of its high specific heat capacity. Therefore, after the shipping container has been warmed up to ambient temperature for cleaning, there is a long duration of down-time (during which the shipping container is cooled). During this down-time, the shipping container is not being used for transporting biological samples.

Accordingly, there exists a need to provide an improved approach for retaining, in particular transporting, cryopreserved samples, which addresses the disadvantages of existing systems listed above, and associated methods.

### SUMMARY

This summary introduces concepts that are described in more detail in the detailed description. It should not be used to identify essential features of the claimed subject matter, nor to limit the scope of the claimed subject matter.

In particular, the present invention relates to a bung as is defined by appended claim 1.

The container described herein used with such a bung may be for retaining one or more cryopreserved samples. As used herein, retaining one or more cryopreserved samples may comprise transporting one or more cryopreserved samples and/or storing one or more cryopreserved samples. In other words, the container may be for retaining one or more cryopreserved samples whether the container is in motion or is static.

Such a container has to have an access for inserting and removing items to be cryogenically stored or transported. In this invention that access is afforded by the bung for closing the opening of the container to form a cryogenic storage apparatus.

### BRIEF DESCRIPTION OF FIGURES

Specific embodiments are described below by way of example only and with reference to the accompanying drawings, in which:
FIG. 1 is a schematic diagram of a cross-sectional view of a container for retaining, in particular transporting, cryopreserved samples without a bung fitted.
FIG. 2 is a schematic diagram of a cross-sectional view of a bung for insulating a container from the ambient environment.
FIG. 3 is a schematic diagram of a cross-sectional view of a container for retaining, in particular transporting, cryopreserved samples with the bung of FIG. 1 fitted.
FIG. 4 is a schematic diagram of a cross-sectional view of an alternative bung to the bung shown in FIG. 2.
FIG. 5 is a schematic diagram of a cross-sectional view of an alternative bung to the bung shown in FIG. 2.
FIG. 6 is an enlarged view of a portion of the bung shown in FIG. 5 attached to a container.
FIG. 7 is an perspective view of the bung shown in FIG. 5.
FIG. 8A is perspective view of a top portion of the bung shown in FIG. 5.
FIG. 8B is an exploded view of a portion of the bung shown in FIG. 5
FIG. 9 is a flowchart of a method of cooling a container for retaining, in particular transporting, cryopreserved samples.
FIG. 10 is a flowchart of a method of preparing a shipping system for retaining, in particular transporting, cryopreserved samples.
FIG. 11 is a schematic diagram of an apparatus for sterilising a container for retaining, in particular transporting, cryopreserved samples.
FIG. 12 is a schematic diagram of an alternative apparatus for sterilising a container for retaining, in particular transporting, cryopreserved samples.
FIG. 13 is a flowchart of a method of sterilising a container for retaining, in particular transporting, cryopreserved samples.
FIG. 14 is a flowchart of an additional method of preparing a shipping system for retaining, in particular transporting, cryopreserved samples.
FIG. 15 is a graph showing the change in temperature over time of a shipping system comprising the bung shown in FIG. 2.
FIG. 16 is a schematic diagram of alternative apparatus for sterilising a container for retaining, in particular transporting, cryopreserved samples.

### DETAILED DESCRIPTION

Implementations of the present disclosure are explained below with particular reference to retaining cryopreserved samples, which are maintained at cryogenic temperatures during transport or storage. It will be appreciated, however, that the devices and methods disclosed herein are also applicable to retaining material at other temperatures (i.e. at non-cryogenic temperatures).

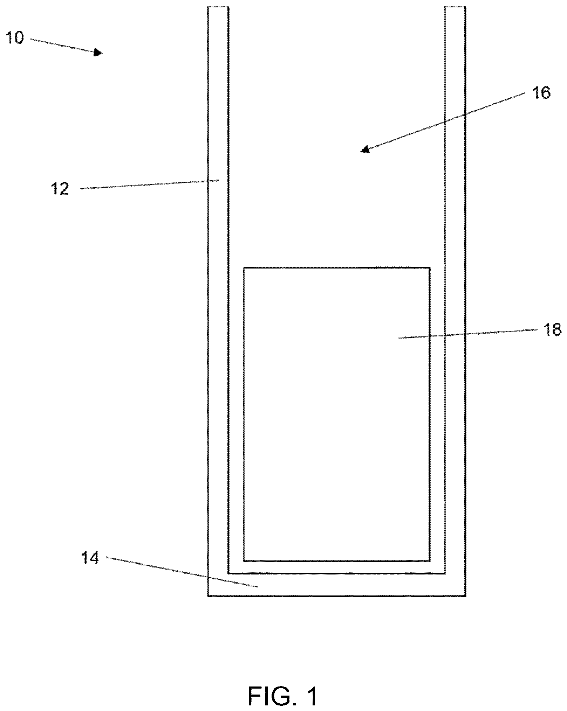

FIG. 1 shows a container for retaining, in particular transporting, cryopreserved samples. As shown in FIG. 1, the container 10 comprises walls 12 and a base 14. The walls 12 and the base 14 are vacuum insulated to provide insulation to the contents of the container 10. The walls 12 and the base 14 define a cavity 16 within the container 10 (i.e. the interior of the container 10).

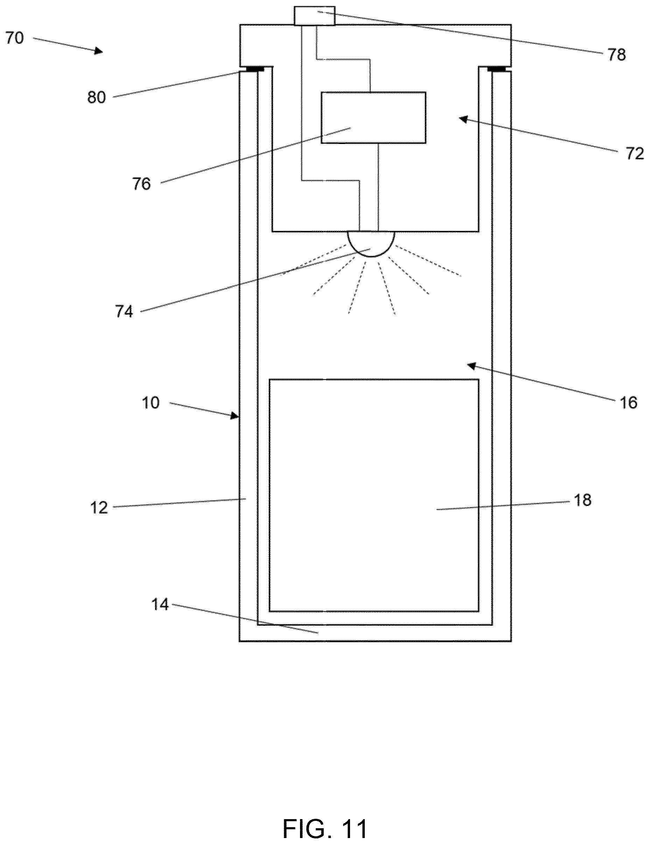

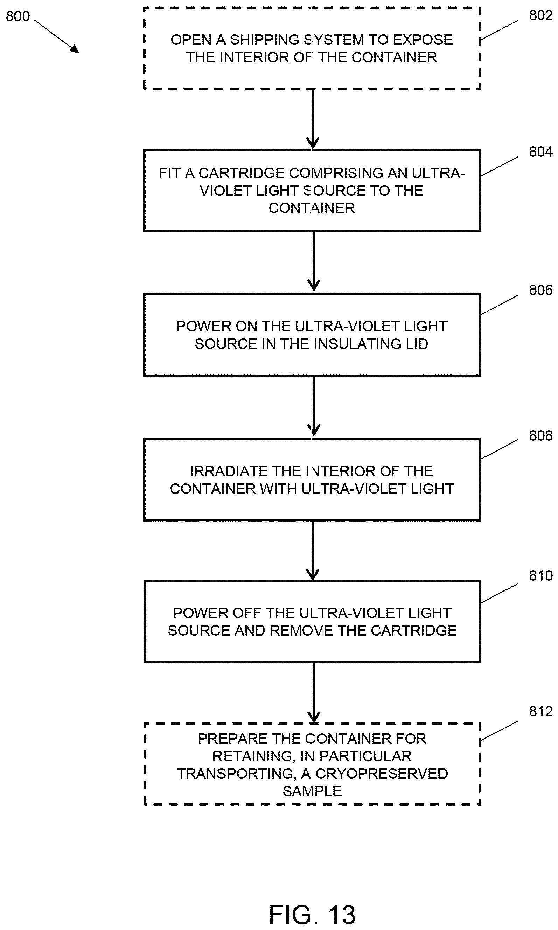

Optionally, to facilitate sterilisation using ultra-violet light (as described in relation to FIG. 11 and FIG. 13 below), the interior of the cavity (i.e. the portions of the walls 12 and/or the base 14 in contact with the cavity 16) may be formed of a material which is reflective to the wavelengths of ultra-violet light used to sterilise the container 10 (for example, a material which is reflective to ultra-violet light having a wavelength of between 100 nm and 300 nm, preferably 275 nm). The walls 12 and/or the base 14 may comprise materials suitable for meeting desired thermal requirements, for example glass fibre or stainless steel. The interior of the cavity 16 (i.e. the portions of the walls 12 and/or the base 14 in contact with the cavity 16) may be coated with a thin reflective layer to assist UV sterilisation.

Returning to the container 10 shown in FIG. 1, a thermal mass 18 is positioned within the cavity 16 of the container 10. The thermal mass 18 includes an opening (not shown) for receiving a cryopreserved sample. The cryopreserved sample is a sample of biological material which is cryogenically frozen (for example, at a temperature of less than -120°C). The container 10 is cooled (for example, to a temperature of less than -120°C) in order to maintain a cryogenic temperature within the container 10 so that the sample is maintained in its cryogenically frozen state.

The thermal mass 18 is formed from a material (such as aluminium) with a high specific heat capacity, meaning that the thermal mass 18 is resistant to changes in temperature. The thermal mass 18 may be formed of a material with a high thermal mass to weight and/or volume ratio, e.g. aluminium, a polymer such as Nylon, or water/ice.

This means that the thermal mass 18 stays cold for a long time after the container 10 itself has been cooled. The thermal mass 18 therefore acts to slow down the rate of increase of the temperature within the cavity 16 and provides passive cooling to the cryopreserved sample received within the opening.

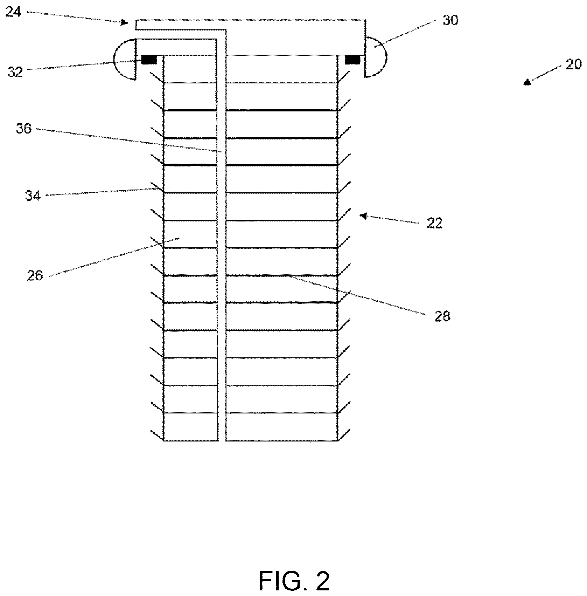

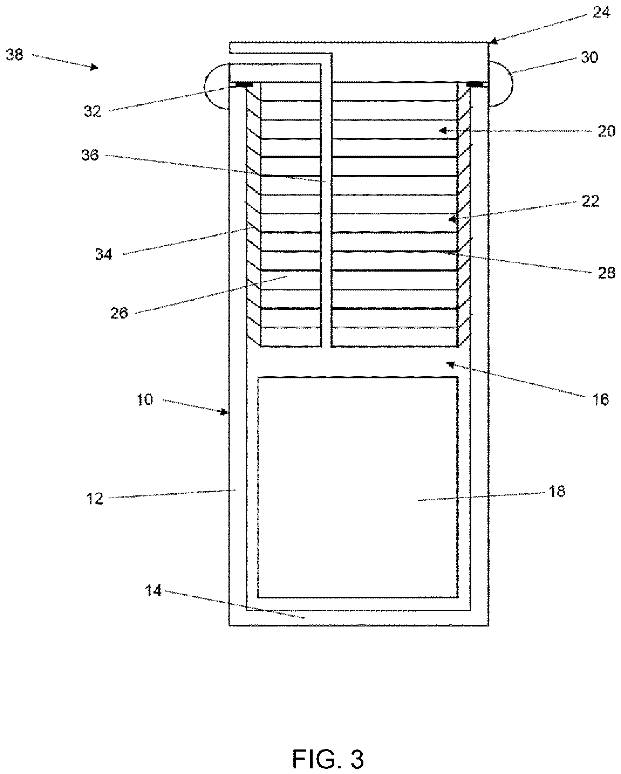

FIG. 2 shows a bung 20 for insulating the interior of the container 10 shown in FIG. 1 from the ambient environment. The bung 20 shown in FIG. 2 is dimensioned so that a portion of the bung 20 fits within the cavity 16 of the container 10. The bung 20 comprises a lower portion 22 and an upper portion 24. The lower portion 22 is arranged to fit within the cavity 16 defined by the walls 12 of the container, as shown in FIG. 3. Returning to FIG. 2, the upper portion 24 has a width which is greater than the width of the cavity 16, so that the upper portion abuts the top of the walls 12 and covers the opening at the top of the cavity 16 when the bung 20 is fitted to the container 10 (as shown in FIG. 3), thereby insulating the interior of the container 10 from the ambient environment.

Referring to FIG. 2 and FIG. 3, the bung 20 comprises a plurality of insulating segments 26 and at least one reflective barrier 28 for reflecting infrared radiation. For example, the lower portion 22 of the bung 20 shown in FIG. 2 comprises fourteen insulating segments 26 with thirteen reflective barriers 28. In one example, the bung 20 may comprise at least 20 insulating segments 26 and reflective barriers 28. Insulation provided by the bung 20 may increase with increasing numbers of insulating segments 26 and/or reflective barriers 28.

Each reflective barrier 28 is arranged between two insulating segments 26 such that the insulating segments 26 and reflective barriers 28 are provided in alternate layers. That is, the insulating segments 26 and reflective barriers 28 are provided in alternate layers along the longitudinal axis of the bung 20, where the longitudinal axis is aligned with the direction in which the bung 20 is fitted to, and/or removed from, the container 10. The top insulating segment 26 in the bung 20 shown in FIG. 2 is attached to the upper portion 24 of the bung 20.

The insulating segments 26 may be formed from an insulating material (i.e. a material with low thermal conductivity and low thermal mass), such as foam. One or more of the insulating segments 26 may comprise closed cell foam, aerogel and/or one or more pockets of vacuum or partial vacuum. The insulating segments 26 provide insulation between the cavity 16 of the container 10 and the ambient environment to slow down the heat transfer from the ambient environment to the cavity 16.

The reflective barriers 28 are formed from a material which reflects infrared radiation (i.e. a material with high reflectivity), such as metallic foil. The reflective barriers 28 reflect infrared radiation from the ambient environment to prevent the infrared radiation from the ambient environment from heating the cavity 16.

The bung 20 further comprises a clamp 30 for attaching the upper portion 24 of the bung 20 to the walls 12 of the container 10 (as shown in FIG. 3). The clamp 30 is mounted to the upper portion 24 of the bung 20. After the bung 20 is fitted to the container 10 (i.e. after the lower portion 22 is fitted within the cavity 16), the clamp 30 is tightened. The clamp 30 tightens the seal at the interface between the upper portion 24 of the bung 20 and the walls 12 of the container 10. Tightening the clamp 30 prevents air flow between the ambient environment and the interior of the cavity 16, meaning that the convective heating provided by the ambient air is reduced.

The bung 20 further comprises a top seal 32 (as best shown in FIG. 2). The top seal 32 is positioned on the underside of the upper portion 24 of the bung 20, as shown in FIG. 2. Therefore, the top seal 32 is compressed between the upper portion 24 of the bung 20 and the top of the walls 12 when the bung 20 is fitted within the cavity 16, as shown in FIG. 3. The top seal 32 also prevents air flow between the ambient environment and the interior of the cavity 16, thereby reducing the convective heating provided by the ambient air.

The bung 20 also comprises flexible seals 34 which are located around the periphery of the lower portion 22 of the bung 20. For ease of attachment, the flexible seals 34 may be mounted to the reflective barriers 28. Alternatively, or in addition, the flexible seals 34 may be integral with or mounted to the insulating segments 26, for example around a perimeter of the insulating segments 26.

The flexible seals 34 are formed of a resilient material which can withstand cryogenic temperatures without deterioration. A suitable material for the flexible seals 34 is rubber. The flexible seals 34 may comprise faces configured to abut the walls 12 and formed of a hard-wearing material, and softer or sprung parts on opposite sides of the faces from the walls 12 and configured to provide compliance/compression of the flexible seals 34.

When the bung 20 is not fitted in the container 10 (i.e. as shown in FIG. 2), the total width of the lower portion 22 of the bung 20 and the flexible seals 34 exceeds the width of the cavity 16 (i.e. the gap between the walls 12 of the container 10). This means that when the bung 20 is fitted to the container 10 (i.e. as shown in FIG. 3), the flexible seals 34 are compressed between the lower portion 22 of the bung 20 and the walls 12 of the container 10, thereby sealing the gap between the lower portion 22 of the bung 20 and the walls 12 of the container 10.

When the bung 20 is removed from the container 10, the flexible seals 34 return to their original shape.

When the bung 20 is fitted to the container 10 (i.e. as shown in FIG. 3), the compressed flexible seals 34 prevent air from flowing between the interior of the cavity 16 of the container 10 and the ambient environment through the gap between the lower portion 22 of the bung 20 and the walls 12 of the container 10.

This means that the flexible seals 34 reduce the convective heating provided by the ambient air.

As noted above, each of the clamp 30, top seal 32 and flexible seals 34 prevent heating from convective air flows between the bung 20 and the walls 12 of the container. Each of these components prevents heating of the sample in the cavity 16 when the container 10 is on its side or upside down. Each of these components also acts to retain cold air within the cavity 16 when the container 10 is on its side or upside down by preventing heat transfer through the top of the container 10 (i.e. the end of the container opposed to the base 14). Therefore, the thermal mass 18 is not only effective when the container 10 is the correct way up, but also when the container 10 is on its side or upside down.

The bung 20 further comprises a vent channel 36. The vent channel 36 passes through the lower portion 22 of the bung 20 and is open to the interior of the cavity 16 when the bung 20 is fitted to the container 10 (as shown in FIG. 3). The vent channel 36 also passes through the upper portion 24 of the bung 20 and is open to the ambient environment (as shown in FIG. 2 and FIG. 3). This means that the vent channel 36 allows a small amount of air to pass between the interior of the cavity 16 and the ambient environment.

As shown in FIG. 2, the vent channel 36 runs vertically through the whole of the lower portion 22 of the bung 20 and vertically through part of the upper portion 24 of the bung 20. The vent channel 36 then runs horizontally between its vertical extent in the upper portion 24 of the bung 20 to the side of the upper portion 24 of the bung 20, which is exposed to the ambient environment.

The vent channel 36 allows air to escape from the cavity 16 to avoid over-pressurisation of the container 10 resulting from expansion of the thermal mass 18 in the cavity 16. To explain, as the thermal mass 18 in the cavity 16 heats up (which will happen over time during transportation or storage of the container 10), the thermal mass 18 expands. The expansion of the thermal mass 18 displaces the air in the cavity 16, meaning that the pressure of the air in the cavity 16 would increase if air was not allowed to escape via the vent channel 36.

The vent channel 36 also allows air to escape from the cavity 16 when the bung 20 is being fitted to the container 10, to avoid over-pressurisation of the container 10. To explain, if air was not allowed to escape through the bung 20, then the action of fitting the bung 20 to the container 10 would increase the pressure of the air within the cavity 16. Allowing the air to escape through the vent channel 36 therefore prevents this build-up of pressure. This means that the bung 20 can be fitted to the container 10 more easily, because the user does not need to apply additional force when inserting the bung 20 into the container 10 to counteract a force applied to the bung 20 by pressurised air in the cavity 16.

Likewise, the vent channel 36 allows air ingress into the cavity 16 when the bung 20 is being removed from the container 10, to avoid generation of a vacuum within the cavity 16. To explain, if air ingress was not allowed through the bung 20, then the action of removing the bung 20 from the container 10 would reduce the pressure of the air within the cavity 16 (thereby generating a vacuum). Allowing air ingress through the vent channel 36 therefore prevents the generation of such a vacuum. This means that the bung 20 can be removed from the container 10 more easily, because the user does not need to apply additional force when removing the bung 20 from the container 10 to counteract a force applied to the bung 20 by the vacuum in the cavity 16.

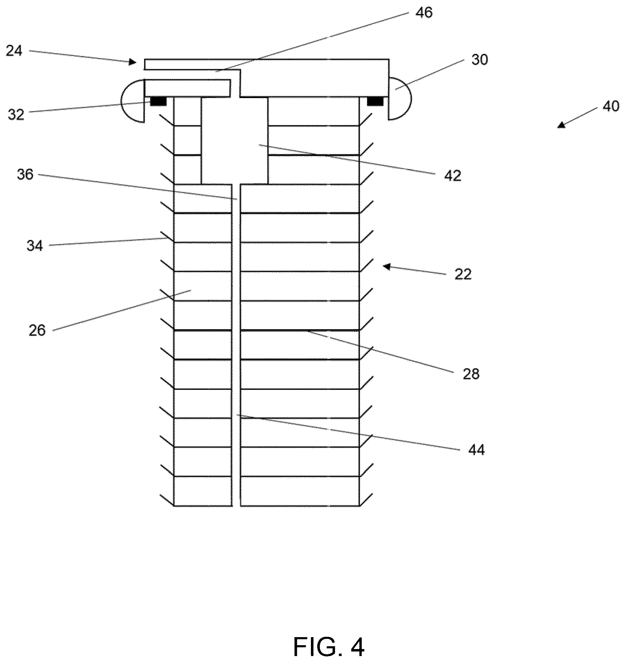

The cross-section of the vent channel 36 is chosen so that it is large enough to permit airflow through the vent channel 36 without getting blocked (e.g. by frost/ice, as explained in more detail in relation to FIG. 4) but narrow enough so that convective heating by air ingress into the cavity 16 via the vent channel 36 is insignificant. For example, the vent channel 36 may have a circular cross-section with a diameter of 6 mm or less. Limiting the diameter of the vent channel 36 limits convective heating by air ingress into the cavity 16. Alternatively, or in addition, the vent channel 36 may be formed in a zig-zag, tortuous or winding shape. This limits convective heating by increasing a path length of the vent channel 36.

Optionally, the vent channel 36 includes one or more valves (not shown in FIG. 2 or FIG. 3) which reduce convection in the vent channel 36 while allowing air in/out when the bung 20 is removed/inserted. The valves may also help control the moisture within the vent channel 36 to prevent the build-up of frost/ice.

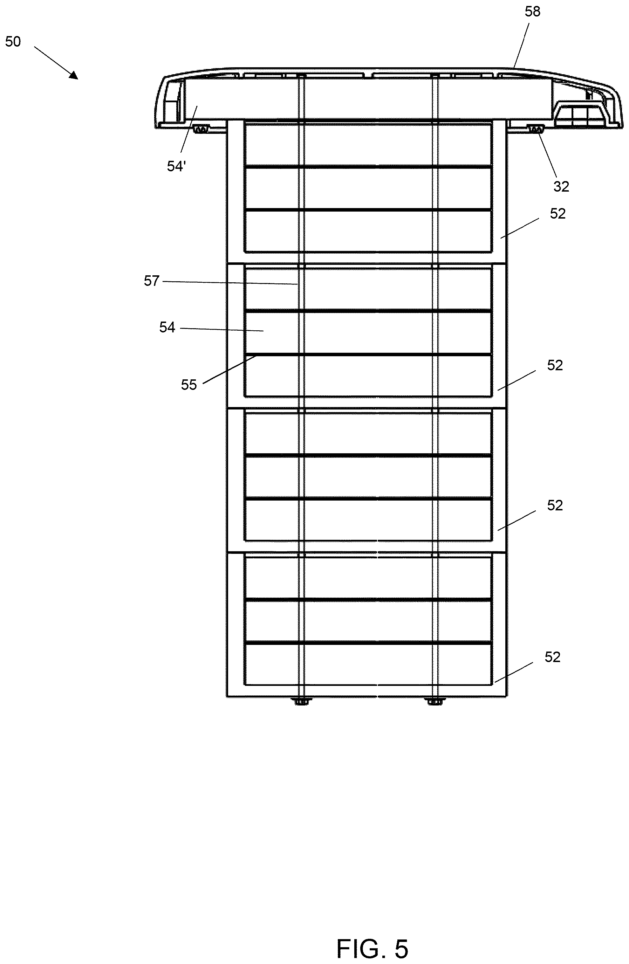

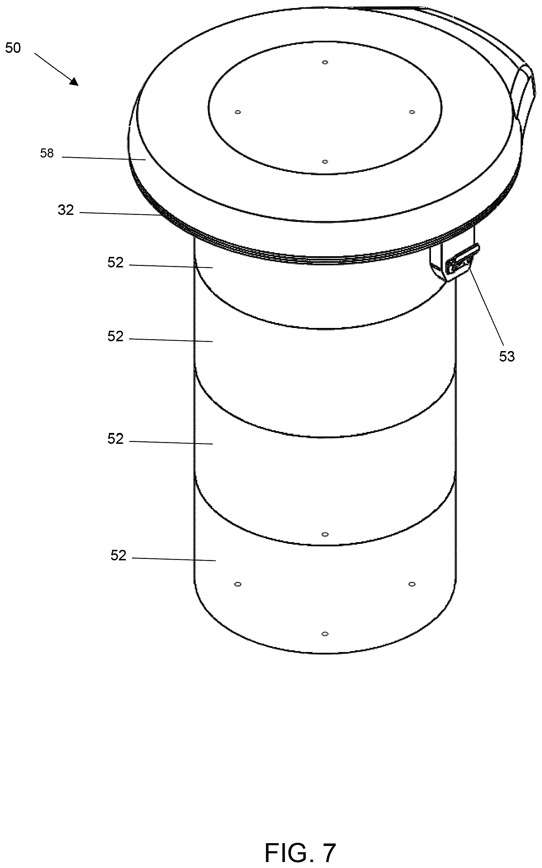

FIG. 5 shows a bung 50 for insulating the interior of the container 10 shown in FIG. 1 from the ambient environment. The bung 50 shown in FIG. 5 is dimensioned so that a portion of the bung 50 fits within the cavity 16 of the container 10. The bung 50 comprises a lower portion and an upper portion. The lower portion is arranged to fit within the cavity 16 defined by the walls 12 of the container. The upper portion has a width which is greater than the width of the cavity 16, so that the upper portion abuts the top of the walls 12 and covers the opening at the top of the cavity 16 when the bung 50 is fitted to the container 10 (similar to the illustration of FIG. 3), as partially shown in FIG. 7, thereby insulating the interior of the container 10 from the ambient environment.

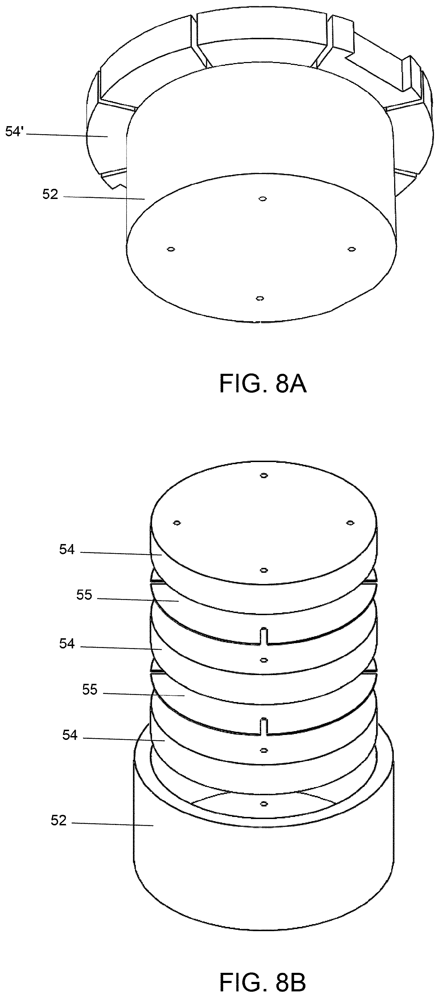

Referring to FIGS. 5-8B, the bung 50 comprises a plurality of chambers 52. Each chamber, as best illustrated by FIGS. 5 and 8B, has a bottom, a side wall, and an open top, creating a cavity (e.g., is in the shape of a bucket), the cavity housing a plurality of insulation segments 54, which are separated by spacers 55. According to the example shown in FIGS. 5 and 8B, each chamber 52 includes three insulation segments 54 and two spacers 55, the spacers 55 being located between adjacent insulation segments 54. However, more or less insulation segments 54 and spacers 55 are contemplated (e.g., using two insulation segments 54 with one spacer 55, using four insulating segments with three spacers 55, etc). In other words, each chamber 52 includes alternate layers of insulating segments 54 and spacers 55. The chambers 52 are stacked on top of one another. As illustrated by FIGS. 5 and 7, the bung 50 includes four chambers in a stacked configuration. However, more or less chambers can be used (e.g., two, three, or more than four).

That is, each chamber 52 includes alternate layers of insulating segments 54 and spacers 55 along the longitudinal axis of the bung 50, where the longitudinal axis is aligned with the direction in which the bung 50 is fitted to, and/or removed from, the container 10. Additionally, reflective barriers 56 (not illustrated) may be placed on the top and bottom surfaces of each of the insulation segments 54. In this way, the bung 50 effectively doubles the number of the infrared radiation barriers (e.g., a bung with two insulation segments 54 and one spacer 55 includes 4 reflective barriers 56). The top insulating segment 54' in the bung 50 shown in FIGS. 5, 6, and 8A is attached to a top surface of the uppermost chamber 52 and located within a lid 58 of the bung 50.

As FIGS. 5, 6, and 8B illustrate, the insulating segments 54 are thicker (i.e., have a greater height) than the spacers 55. According to embodiments, the insulating segments 54 have a thickness between 75-10 mm, while the spacers have a thickness between 75-1 mm. In one illustrative example, the insulating segments 54 are approximately 25mm thick, while the spacers 55 are approximately 1mm thick. It is to be noted that in alternative embodiments, the spacers 55 and insulating elements 54 can have the same thickness or the spacers 55 can be thicker than the insulating elements 54. Additionally, the thickness of the spacers 55 and/or insulating elements 54 may vary along the length of the bung 50. In one example, the thickness of the insulating elements 54 decreases along the length of the bung 50, such that the thickness of the insulating elements 54 is at a minimum value for the insulating element 54 that is farthest away from the lid 58.

As further shown in FIG. 5, at least one rod 57 is located within and through the plurality of chambers 52 (and thus through the corresponding insulating segments 54, spacers 55, and reflective barriers 56). The rods 57 provide structural support for each of the chambers 52 and to ensure that the plurality of chambers 52 (and thus the corresponding insulating segments 54, spacers 55, and reflective barriers 56) are fixed together. The rods can be made of any suitable material that provides sufficient strength to keep the chambers 52 in their stacked configuration (e.g., made from a polymer, etc.). Preferably, the rods are thin and made from a material with low thermal conductivity. Alternatively, the rods 57 may be omitted and the stacked chambers can be fixed to one another through other means (e.g., gluing).

The chambers may be formed from an insulating material (i.e. a material with low thermal conductivity and low thermal mass), such as foam. One or more of the insulating segments 54 may comprise closed cell foam, aerogel and/or one or more pockets of vacuum or partial vacuum. In preferred embodiments, the containers are formed from an insulating material that is easily machinable, such as a low-density Styrofoam.

The insulating segments 54 may be formed from an insulating material (i.e. a material with low thermal conductivity and low thermal mass), such as foam. One or more of the insulating segments 54 may comprise closed cell foam, aerogel and/or one or more pockets of vacuum or partial vacuum. The insulating segments 54 provide insulation between the cavity 16 of the container 10 and the ambient environment to slow down the heat transfer from the ambient environment to the cavity 16. Similarly, the spacers 55 may also be made of an insulating material (i.e. a material with low thermal conductivity and low thermal mass), such as foam. One or more of the spacers 55 may comprise closed cell foam, aerogel and/or one or more pockets of vacuum or partial vacuum.

The reflective barriers 56 are formed from a material which reflects infrared radiation (i.e. a material with high reflectivity), such as metallic foil. The reflective barriers 56 reflect infrared radiation from the ambient environment to prevent the infrared radiation from the ambient environment from heating the cavity 16.

The bung 50 further comprises a clamp 53 for attaching the lid 58 of the bung 50 to the walls 12 of the container 10 (as shown in FIG. 7). The clamp 53 is mounted to or a part of the lid 58. After the bung 50 is fitted to the container 10, the clamp 53 is tightened. The clamp 53 tightens the seal at the interface between the upper portion of the bung 50 and the walls 12 of the container 10. Tightening the clamp 53 prevents air flow between the ambient environment and the interior of the cavity 16, meaning that the convective heating provided by the ambient air is reduced.

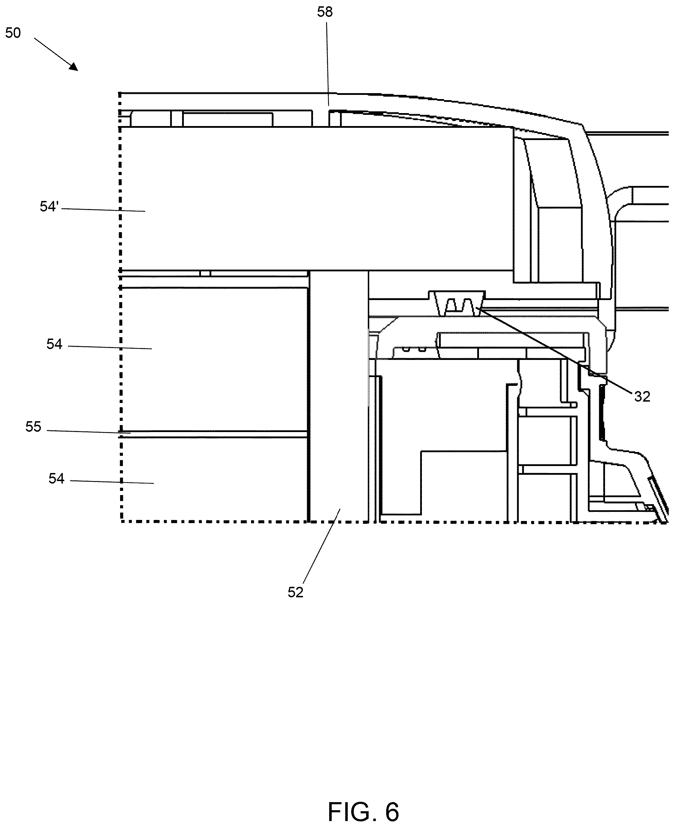

The bung 50 further comprises a top seal 32 (as best shown in FIGS. 5-7). The top seal 32 is positioned on the underside of the lid 58 of the bung 50, as shown in FIGS. 6 and 7. Therefore, the top seal 32 is compressed between the upper portion of the bung 50 and the top of the walls 12 when the bung 50 is fitted within the cavity 16. The top seal 32 also prevents air flow between the ambient environment and the interior of the cavity 16, thereby reducing the convective heating provided by the ambient air.

As described in more detail below with reference to FIG. 11, the bung 20, 40, 50 may optionally comprise an ultra-violet light source (not shown in FIG. 2, 4 or 5).

As noted above, the bung 20, 40, 50 is fitted to the container 10 shown in FIG. 1. Together, the container 10 and the bung 20, 40, 50 form a shipping system 38. The shipping system 38 therefore comprises a vacuum-insulated container 10 comprising a thermal mass 18, and a bung 20, 40, 50 for insulating the interior of the container 10 from the ambient environment.

As noted above, the thermal mass 18 acts to slow down the rate of increase of the temperature within the cavity 16 and provides passive cooling to the cryopreserved sample received within the opening. Consequently, the container 10 allows cryopreserved samples to be retained, in particular transported, without requiring the use of liquid nitrogen, owing to the passive cooling provided by the thermal mass 18. Given that no liquid nitrogen is used in the container 10, the shipping system 38 does not need to provide for venting of evaporated liquid nitrogen. This means that a bung (such as the bung 20, 40, 50 shown in FIGS. 2, 4 and 5) can be used to insulate the interior of the container 10 from the ambient environment.

The bung 20, 40, 50 insulates the interior of the container 10 to prevent heat transfer to the cavity 16 of the container 10 from the ambient environment. The insulation provided by the bung 20, 40, 50 means that the shipping system 38 is able to maintain the cryopreserved sample at cryogenic temperatures, even when the shipping system 38 is positioned on its side or upside down. That is, the bung 20, 40, 50 acts to retain cold air within the cavity 16 of the container 10 when the shipping system 38 is on its side or upside down by preventing heat transfer through the top of the container 10 (i.e. the end of the container opposed to the base 14).

FIG. 4 shows an alternative bung 40 to the bung 20 shown in FIG. 2. The bung 40 includes all of the components of the bung 20 shown in FIG. 2, which are identified using the same reference numerals as used in FIG. 2. The bung 40 can be fitted to the container 10 shown in FIG. 1 in the same way as the bung 20 shown in FIG. 2. In addition to the components of the bung 20 of FIG. 2, the bung 40 shown in FIG. 4 includes a chamber 42 in the path of the vent channel 36.

The chamber 42 is located towards the top of the portion of the vent channel 36 that runs through the lower portion 22 of the bung 40. The chamber 42 is formed by making holes in some of the insulating segments 26 and reflective barriers 28.

For ease of reference, the vent channel 36 is shown in FIG. 4 with two sections: a lower part 44 and an upper part 46. The lower part 44 of the vent channel 36 extends between the chamber 42 and the cavity 16 (when the bung 20 is fitted to the container 10). The upper part 46 of the vent channel 36 extends between the chamber 42 and the ambient environment.

Air flows through the lower part 44 of the vent channel 36 in the lower portion 22 of the bung and into the chamber 42 formed by the holes in the insulating segments 26 and reflective barriers 28. Air then flows from the chamber 42 to the ambient environment through the upper part 46 of the vent channel.

The chamber 42 is formed at a location in the path of the vent channel 36 which is susceptible to the build-up of frost/ice. Frost/ice builds up in the vent channel 36 at the point at which the air in the vent channel 36 is approximately 0°C. The formation of frost/ice occurs in a specific zone of the vent channel 36. The chamber 42 is formed in the zone where frost/ice accumulates (i.e. at the portion of the vent channel 36 where the temperature of the air in the vent channel 36 is 0°C). The chamber 42 therefore has an upper extent in communication with the upper part 46 of the vent channel 36 where the temperature of the air in the vent channel 36 is above 0°C, and a lower extent in communication with the lower part 44 of the vent channel 36 where the temperature of the air in the vent channel 36 is below 0°C. The portion of the vent channel 36 where the temperature of the air is approximately 0°C is in the lower portion 22 of the bung 40 when the container interior is cooled to a cryogenic temperature. In use, ambient temperatures may typically be in a range of 5°C to 30°C. In use, the end of the bung 20 within the cavity 16 may typically be at a temperature in a range of - 196°C to -120°C. Within such temperature ranges, the region in the vent channel 36 in which the temperature is approximately 0°C in use is relatively narrow, and the chamber 42 can be located to coincide with this region.

That is, the chamber 42 is formed in the lower portion 22 of the bung 40. Specifically, the chamber 42 is located in the lower portion 22 of the bung and is in contact with the upper portion 24 of the bung 40. As shown in FIG. 4, the upper portion 24 of the bung 40 forms one wall of the chamber 42.

The chamber 42 has a greater cross-sectional area than the vent channel 36, meaning that any frost/ice which forms in the chamber 42 is less likely to cause a blockage in the vent channel 36.

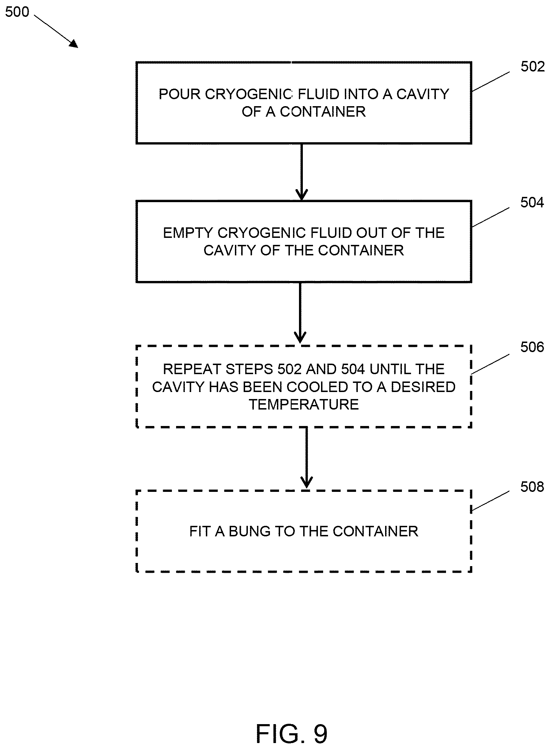

FIG. 9 is a flowchart of a method 500 of cooling a container for retaining, in particular transporting, cryopreserved samples, such as the container 10 shown in FIG. 1.

At step 502, a cryogenic fluid (in this example, liquid nitrogen) is poured into a cavity of the container. Heat is transferred from a thermal mass located within the cavity to the liquid nitrogen in the cavity. The liquid nitrogen therefore cools the thermal mass in the cavity. Optionally, before the liquid nitrogen is poured into the cavity, a bung (such as the bung 20, 40 50 shown in FIGS. 2, 4, and 5) is removed from the container.

After the thermal mass has been cooled by the nitrogen (for example, after a period of approximately 0.5 hours to 1 hour), the liquid nitrogen is emptied out of the cavity at step 504.

Pouring the liquid nitrogen into the cavity allows the cavity to be pre-cooled. Precooling the cavity reduces the length of time that an alternative source of cooling (such as a heat engine) is required to be used in order to cool the interior of the container to a cryogenic temperature.

Optionally, at step 506, steps 502 and 504 are repeated to further lower the temperature within the cavity. For example, these steps may be repeated if the thermal mass has not been cooled down to the desired temperature. That is, at step 506, additional liquid nitrogen is poured into the cavity to cool the thermal mass and subsequently poured out of the cavity after a period of time. Further cycles of pouring liquid nitrogen into the cavity and emptying the liquid nitrogen from the cavity 16 may be carried out until the cavity has been cooled to a desired temperature. For example, further cycles may be repeated until the thermal mass has been cooled to a cryogenic temperature. Repeating these cycles further reduces (or even eliminates) the length of time that an alternative cooling source is required to be used. In some examples, an adequate quantity of the cryogenic fluid may be used at step 502 such that the temperature of the cavity 16 reaches a desired temperature without repetition of steps 502 and 504 (i.e. without step 506).

In some examples, cooling of the cavity 16 may be achieved using a heat engine such as a cryocooler (e.g. a Stirling cryocooler), i.e. without addition or removal of a cryogenic fluid. In other words, step 502 may be replaced with a step of fitting a heat engine to the container 10 to remove heat from the cavity 16. Similarly, step 504 may be replaced with a step of removing the heat engine from the container 10.

Optionally, at step 508, a bung (such as the bung 20, 40, 50 shown in FIGS. 2, 4, 5) is fitted to the container to prevent heat transfer from the ambient environment to the cooled thermal mass. Fitting a bung to the container slows down the rate of temperature increase within the container. The bung may therefore be fitted to the container until a cryopreserved sample is ready to be loaded into the container.

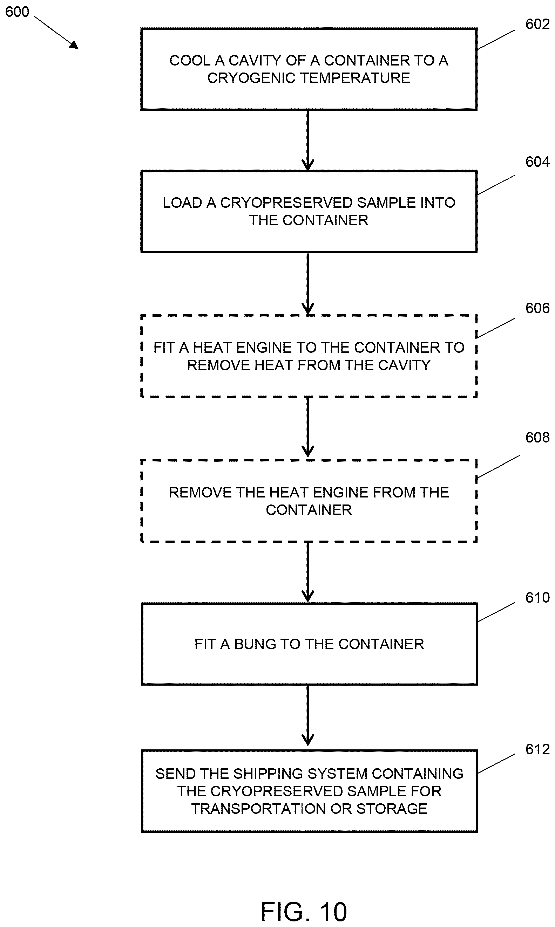

FIG. 10 is a flowchart of a method 600 of preparing a shipping system for retaining, in particular transporting, cryopreserved samples, such as the shipping system 38 shown in FIG. 3 or a shipping system implementing the bung of FIGS. 4, 5, and 11.

At step 602, a cavity of a container for retaining, in particular transporting, cryopreserved samples (such as the container 10 shown in FIG. 1) is cooled to a cryogenic temperature. For example, the container interior may be cooled using a heat engine (e.g., a Stirling cryocooler). Alternatively, the container interior may be cooled by carrying out the steps of the method 500 described with reference to FIG. 9.

At step 604, a cryopreserved sample is loaded into the container. For example, the cryopreserved sample may be placed within the opening in the thermal mass 18 of the container 10 of FIG. 1.

Optionally, at step 606, a heat engine (in this example, a Stirling cryocooler) is fitted to the container 10 to remove heat from the cavity, so that the cryopreserved sample is maintained at the appropriate cryogenic temperature. The Stirling cryocooler may be mounted to the container 10 until the cryopreserved sample is to be transported to or stored in a different location. The Stirling cryocooler may also be mounted to the container 10 to reverse any temperature increase within the container 10 which has occurred during loading of the cryopreserved sample into the container.

If a heat engine such as a Stirling cryocooler has been mounted to the container 10 to maintain the cryopreserved sample at the cryogenic temperature, then it is removed at step 608 prior to transporting or storing the cryopreserved sample.

At step 610, a bung (such as the bung 20, shown in FIGS. 2, 4, 5, and 11) is fitted to the container 10 to insulate the interior of the container from the ambient environment and prevent heat transfer from the ambient environment to the cryopreserved sample. Fitting the bung to the container forms the shipping system 38.

At step 612, the shipping system 38 containing the cryopreserved sample is sent for transportation or storage.

FIG. 11 shows apparatus 70 for sterilising a container for retaining, in particular transporting, cryopreserved samples, such as the container 10 shown in FIG. 1. The container 10 shown in FIG. 11 includes all of the components of the container 10 shown in FIG. 1 (i.e. the walls 12, base 14, cavity 16 and thermal mass 18). The apparatus 70 also includes a cartridge 72. The cartridge 72 is dimensioned so that a portion of the cartridge 72 is arranged to fit within the open end of the container 10 while a separate portion of the cartridge 72 is arranged to abut the ends of the container walls 12 at the open end of the container 10.

The cartridge 72 includes an ultra-violet light source 74, such as a 60W fluorescent tube bulb light. Alternatively, the ultra-violet light source 74 may be a single LED (for example, a 5W LED) or an array of LEDs (for example, three 2W LEDs). The ultra-violet light source 74 emits ultra-violet light in the UVC range or at the lower end of the UVB range.

For example, the ultra-violet light source 74 may emit ultra-violet light with a wavelength of between 100 nm and 300 nm. In a specific example, the ultra-violet light source 74 may emit ultra-violet light having a wavelength of 295 nm.

In the apparatus shown in FIG. 11, the ultra-violet light source 74 is powered by a battery 76 located in the cartridge 72. The ultra-violet light source 74 is controlled using a switch 78 which is accessible when the cartridge 72 is inserted into the open end of the container 10. Alternatively, or in addition, a power supply unit may be mounted on the container 10. The power supply unit is connectable to the mains. The cartridge 72, when mounted on the container 10, may form an electrical connection with the power supply unit. This electrical connection may be via pogo pins, via a connection typically found on a kettle, or via any other suitable connector, preferably enabling forming and breaking of the electrical connection to be implemented easily. Alternatively, or in addition, the cartridge 72 may be connected directly to the mains.

Fitting the cartridge 72 to the container 10 allows the interior of the cavity 16 of the container 10 to be irradiated with ultra-violet light. By irradiating the cavity 16 of the container 10 with ultra-violet light, the cavity 16 is sterilised. Sterilising the container 10 prevents contamination of biological samples from a number of contaminants. For example, the biological sample is prevented from being contaminated by bacteria, viruses, fungi and other microbes, as well as DNA, RNA and cell fragments from biological samples that have previously been retained in the container 10.

The cartridge 72 may contain one or more insulating segments (not shown in FIG. 11) to prevent or slow down heat transfer between the ambient environment and the interior of the cavity 16 while the cavity 16 is being sterilised. In addition, the cartridge 72 comprises a seal 80 located on the underside of the portion of the cartridge 72 that abuts the ends of the walls 12 of the container 10. Optionally, the cartridge 72 may include some of the components of the bung 20 shown in FIG. 2 (or the bung 40, 50 shown in FIGS. 4 and 5), in order to insulate the container interior from the ambient environment while the container interior is being sterilised. For example, the cartridge 72 includes one or more of the insulating segments 26, reflective barriers 28, clamp 30, flexible seals 34, vent channel 36 and chamber 42 shown in FIG. 2 and FIG. 4, or the chambers 52, insulating segments 54, spacers 55, reflective barriers 56, lid 58, flexible seals 34 shown in FIG. 5. As a further example, the cartridge 72 may have the same construction as the bung 20 shown in FIG. 2 (or the bung 40, 50 shown in FIGS. 4 and 5) and further including the ultra-violet light source 74 (and optionally the battery 76 and switch 78). Alternatively, the cartridge 72 may be merged or formed integrally with the heat engine.

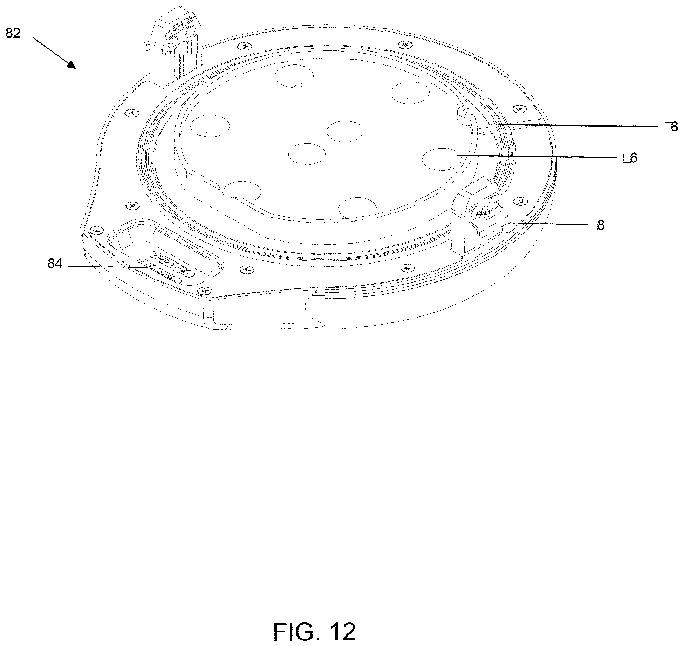

FIG. 12 illustrates the underside of cartridge 82, which is a variation of the cartridge 72 just described. Cartridge 82 includes at least one clamp 88, seal 80, and at least one ultra-violet light source 86, to carry out the same functions as described above with regard to cartridge 72. In this variation, however, multiple ultra-violet light sources 86 are located within a central region of the cartridge 82, as illustrated. In the specific embodiment depicted, eight ultra-violet light sources 86 are integrated into the cartridge 82. Specifically, two ultra-violet light sources 86 are located near the approximate center, while another six ultra-violet light sources 86 are located in circumferential or radial pattern further towards the periphery of the cartridge 82. The circumferentially or radially located ultra-violet light sources 86 may be angled such that they direct light towards the center of the container 10 in use. In one specific example, the six ultra-violet light sources 86 may be angled inward with an inclination angle of approximately 30 degrees, which provides an optimal focus of light on the container 10.

Similar to the embodiment of FIG. 11, cartridge 82 is dimensioned so that a portion of the cartridge 82 is arranged to fit within the open end of the container 10 while a separate portion of the cartridge 82 is arranged to abut the ends of the container walls 12 at the open end of the container 10.

The cartridge 82 includes ultra-violet light sources 86, such as LEDs (for example, 5W LEDs or 2W LEDs). The ultra-violet light sources 86 emits ultra-violet light in the UVC range or at the lower end of the UVB range.

For example, the ultra-violet light sources 86 may emit ultra-violet light with a wavelength of between 100 nm and 300 nm. In a specific example, the ultra-violet light sources 86 may emit ultra-violet light having a wavelength of 295 nm.

In the apparatus shown in FIG. 12, the ultra-violet light sources 86 are powered by a battery located in the cartridge 82. Alternatively, or in addition, a power supply unit may be mounted on the container 10. The power supply unit is connectable to the mains. The cartridge 82, when mounted on the container 10, may form an electrical connection with the container 10, via connector 84. This electrical connection may be via pogo pins, via a connection typically found on a kettle, or via any other suitable connector, preferably enabling forming and breaking of the electrical connection to be implemented easily. In one specific example, connector 84 includes ground and voltage connections and a serial communication interface (e.g., an RS232) allowing for bi-directional communication between electrical components in the cartridge 82 and electrical components within the container 10, which will be further discussed below.

Fitting the cartridge 82 to the container 10 allows the interior of the cavity 16 of the container 10 to be irradiated with ultra-violet light. By irradiating the cavity 16 of the container 10 with ultra-violet light, the cavity 16 is sterilised. Sterilising the container 10 prevents contamination of biological samples from a number of contaminants. For example, the biological sample is prevented from being contaminated by bacteria, viruses, fungi and other microbes, as well as DNA, RNA and cell fragments from biological samples that have previously been retained in the container 10.

The cartridge 82 may contain one or more insulating segments (not shown in FIG. 12) to prevent or slow down heat transfer between the ambient environment and the interior of the cavity 16 while the cavity 16 is being sterilised. In addition, the cartridge 82 comprises a seal 88 located on the underside of the portion of the cartridge 82 that abuts the ends of the walls 12 of the container 10. Optionally, the cartridge 82 may include some of the components of the bung 20 shown in FIG. 2 (or the bung 40, 50 shown in FIGS. 4 and 5), in order to insulate the container interior from the ambient environment while the container interior is being sterilised. For example, the cartridge 82 includes one or more of the insulating segments 26, reflective barriers 28, clamp 30, flexible seals 34, vent channel 36 and chamber 42 shown in FIG. 2 and FIG. 4, or the chambers 52, insulating segments 54, spacers 55, reflective barriers 56, lid 58, flexible seals 34 shown in FIG. 5. As a further example, the cartridge 82 may have the same construction as the bung 20 shown in FIG. 2 (or the bung 40, 50 shown in FIGS. 4 and 5) and further including the ultra-violet light sources 86 (and optionally the battery 76. Alternatively, the cartridge 82 may be merged or formed integrally with the heat engine.

Once connected to the container 10, cartridge 72, 82 is in electrical communication with the container 10 (e.g., through connector 84). In embodiments, each of container 10 and cartridge 72, 82 includes at least one sensing element and at least one controller. As non-limiting examples, the container may include sensing elements that measure temperature (e.g., a thermistor) and light (e.g., a UV sensor for measuring how much light the cartridge emits), while the cartridge 72, 82 may include sensing elements to measure voltage, current, and the like. The controller in each of the container 10 and cartridge 72, 82 may include at least one memory for recording signals coming from the sensing elements and for recording calculations made by the processor(s) (e.g., derived a power output based on the sensed voltage and current output). The memory units may each also store a unique identification code, so that each cartridge and each container can be individually identified. Still further, each of the container 10 and cartridge 72, 82 may include GPS system so that the location of each can be monitored.

Each of the container 10 and cartridge 72, 82 may also include a transceiver for wirelessly transmitting information stored in the memory of their respective controllers. The transceivers can transmit the information to a remote location so that the information can be recorded and monitored remotely. By way of example, a central monitoring station may receive the GPS information and/or the sensed and/or calculated information stored in the container 10 and cartridge 72, 82 in order to have real-time information regarding the container 10, the thermal mass 18, and the UV sterilization procedure that the container 10 underwent.

By insulating the cavity 16 against the ambient environment, the container 10 can be sterilised while a cryogenic temperature is maintained in the cavity 16. This means that the requirement to cool the container 10 after sterilisation using the ultra-violet light source 74, 86 may be reduced or even eliminated.

The reduced amount of time required to cool the container 10 after sterilisation in turn reduces the amount of time required to prepare the container 10 for use in retaining, in particular transporting, a subsequent cryopreserved sample, meaning that the down-time of the container 10 in between transports or storage is reduced.

In addition, if the ultra-violet light source includes features which act to insulate the interior of the container 10 from the ambient environment (e.g. the features of the bungs shown in FIG. 2 FIG. 4, and FIG. 5), then the interior of the cavity 16 can be sterilised while the cryopreserved sample is being transported or stored.

In particular, including the ultra-violet light source in the bung used to insulate the interior of the container 10 from the ambient environment would eliminate the requirement to remove the bung in order to fit a separate device for sterilising the container 10. Integrating the ultra-violet light source into the bung used to insulate the interior of the container 10 from the ambient environment also seals the container 10 so that the sterility of the container 10 is maintained after the interior of the container 10 has been irradiated with ultra-violet light.

If the interior of the cavity 16 is sterilised while the cryopreserved sample is being transported or stored, then the cryopreserved sample may be held within a vial or receptacle which is opaque to ultra-violet light, or the cryopreserved sample may be otherwise shielded from the ultra-violet light from the ultra-violet light source. Shielding the cryopreserved sample from the ultra-violet light ensures that the cryopreserved sample is not damaged by the ultra-violet radiation.

Alternatively, if the cryopreserved sample was a sample that required sterilisation (such as a blood product), then the cryopreserved sample could be sterilised at the same time as the cavity 16 if the sample was exposed to the ultra-violet light within the cavity 16.

FIG. 13 is a flowchart of a method 800 of sterilising a container for retaining, in particular transporting, cryopreserved samples, such as the container 10 shown in FIG. 1. The container 10 may be a part of a shipping system, such as the shipping system 38 shown in FIG. 3 (or the shipping system 38 that includes the bung of FIG. 4 or 5). The interior of the container 10 of the shipping system may be insulated from the ambient environment using a bung (such as the bung 20 shown in FIG. 2, the bung 40 shown in FIG. 4, or the bung 50 shown in FIG. 5) or may be coupled to a Stirling cryocooler. The method 800 may be carried out using the apparatus 70 shown in FIG. 11.

Optionally, at step 802, if a bung or cryocooler has been fitted to the container to form a shipping system, then the shipping system is opened to expose the interior of the container. Opening the container may therefore comprise removing a bung or Stirling cryocooler or any other device which is fitted to the open end of the container.

At step 804, a cartridge comprising an ultra-violet light source is fitted to the container so that the open end of the container is closed using the cartridge. By fitting the cartridge comprising the ultra-violet light source to the container, the ultra-violet light source is arranged to irradiate the interior of the container.

At step 806, the ultra-violet light source in the cartridge is activated so that the interior of the container is irradiated with ultra-violet light.

At step 808, the interior of the container is irradiated with ultra-violet light for a sufficient length of time to ensure that the interior of the container is sterilised. For example, the interior of the container may be irradiated with ultra-violet light for between 30 and 60 minutes.

At step 810, the ultra-violet light source is deactivated and the cartridge is removed from the container.

Optionally, at step 812, the container is prepared for retaining, in particular transporting, a cryopreserved sample. For example, the container may be prepared by carrying out the steps of the method 600 described with reference to FIG. 10. As the container can be sterilised while a cryogenic temperature is maintained in the cavity of the container, the requirement to cool the container at step 602 of the method 600 may be reduced or eliminated.

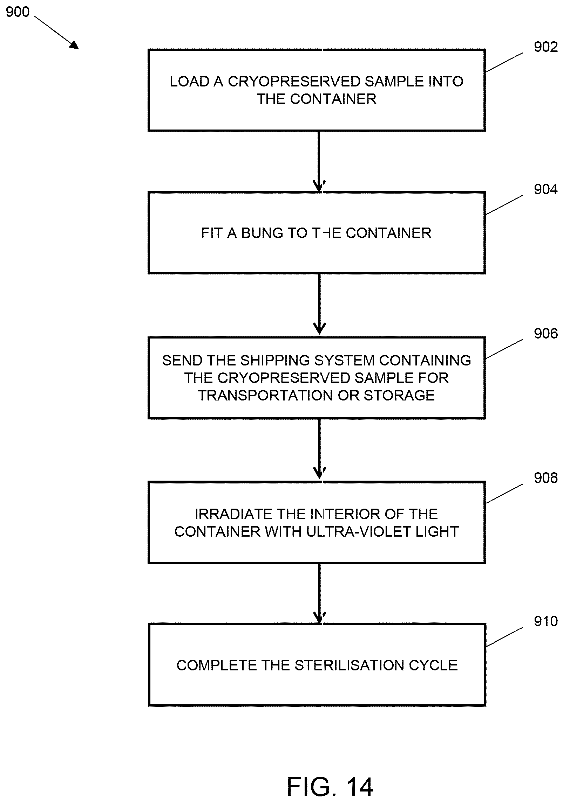

FIG. 14 is a flowchart of an additional method 900 of preparing a shipping system for retaining, in particular transporting, cryopreserved samples, such as the shipping system 38 shown in FIG. 3 (or the shipping system 38 that includes the bung of FIG. 4 or 5). The shipping system may comprise a container (such as the container 10 shown in FIG. 1) and a bung (such as the bung 20 shown in FIG. 2, the bung 40 shown in FIG. 4, or the bung shown in FIG. 5).

At step 902, a cryopreserved sample is loaded into the container. For example, the cryopreserved sample may be placed within an opening in the container 10. Loading the cryopreserved sample into the container may comprise locating the cryopreserved sample within a receptacle which is opaque to ultra-violet light.

At step 904, a bung is fitted to the container to insulate the interior of the container from the ambient environment and prevent heat transfer from the ambient environment to the cryopreserved sample. The bung and/or a component of the container may comprise an ultra-violet light source. Fitting the bung to the container forms the shipping system.

At step 906, the shipping system containing the cryopreserved sample is sent for transportation or storage.

At step 908, the interior of the container is irradiated using ultra-violet light to sterilise the container. The ultra-violet light may have a wavelength of between 100 nm and 300 nm (for example, about 265 to 275 nm). The interior of the container may be sterilised using an ultra-violet light source located in the bung used to insulate the interior of the container from the ambient environment. Alternatively or additionally, the walls and/or the base of the container (or some other component of the container, such as the thermal mass 18 of the container 10 shown in FIG. 1) may comprise an ultra-violet light source which is used to irradiate the interior of the container. Sterilising the container using ultra-violet light may therefore comprise activating an ultra-violet light source located in the bung and/or in a component of the container.

The interior of the container may be irradiated with ultra-violet light for between 30 and 60 minutes (for example, if the container is only being sterilised once during transport or storage) or for a period of approximately 10 minutes (for example, if the container is to be sterilised each day during transport or storage of the cryopreserved sample). Sterilising the container for a short period of time each day during transport or storage of the cryopreserved sample may ensure that the sample does not become contaminated during transport or storage.

At step 910, the sterilisation cycle is completed. Completing the sterilisation cycle may comprise deactivating an ultra-violet light source located in the bung and/or in a component of the container.

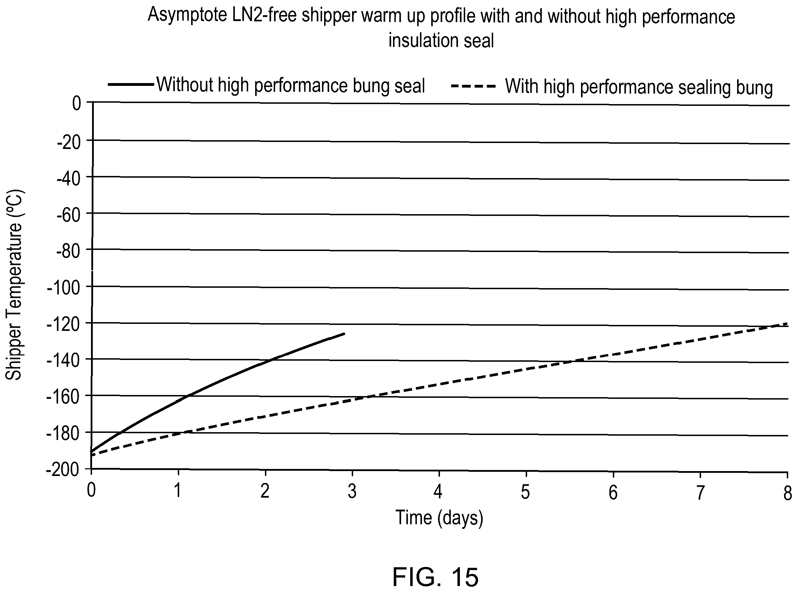

FIG. 15 is a graph showing the change in temperature over time of a shipping system comprising the bung shown in FIG. 2 (lower line), in comparison with the change in temperature over time of a shipping system for which no bung was in place (upper line).

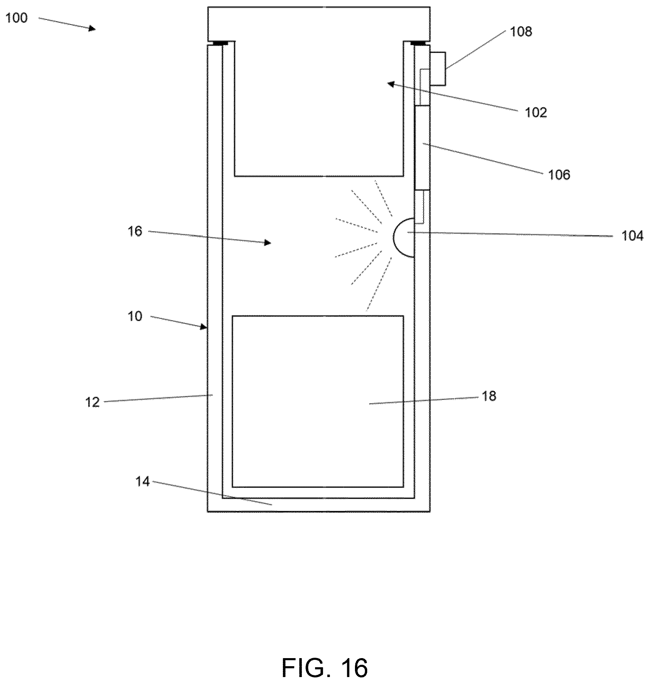

It can be seen from FIG. 16 that fitting the bung shown in FIG. 2 to a container provides a longer period of time over which the container interior is at a cryogenic temperature. The cryogenic zone is between -200°C and -120°C. Fitting the bung shown in FIG. 2 to a container allows the container interior to be maintained at a cryogenic temperature for 8 days, whereas a container without the bung provides only 3 days at a cryogenic temperature.

Fitting the bung shown in FIG. 2 to the container therefore allows cryopreserved samples to be transported and/or stored for longer durations without the cryopreserved sample being damaged by exposure to temperatures outside the cryogenic zone. Allowing cryopreserved samples to be retained, in particular transported, for longer durations increases the distance over which cryopreserved samples may be transported, thereby increasing the population of patients that may be treated using cryopreserved samples from a particular source.

Variations or modifications to the systems and methods described herein are set out in the following paragraphs.

The apparatus 70, 82 shown in FIGS. 11 and 12 has been described in relation to sterilisation of a container 10 comprising a thermal mass 18. As explained above, the thermal mass 18 provides passive cooling to the sample held within the container 10 by slowing down the rate of temperature increase within the cavity 16 of the container 10. However, the methods and apparatus described herein are not limited to the sterilisation of containers comprising thermal masses.

That is, the methods and apparatus described herein may be used to sterilise a container in which the sample held within the container is cooled using other means. In particular, an ultra-violet light source may be used to sterilise a shipping system in which a heat engine such as a Stirling cryocooler is used to provide cooling to the sample held within the container.

In a shipping system in which a heat engine (such as a Stirling cryocooler) is used to maintain the sample at cryogenic temperatures, the Stirling cryocooler may be located on a lid which can be fitted to the container, and a heat exchanger may be attached to the Stirling cryocooler such that the heat exchanger is located within the cavity of the container when the lid is fitted to the container.

The container may comprise liquid nitrogen (or another working fluid) which provides cooling to the cryopreserved sample. The Stirling cryocooler removes heat from the container, meaning that the liquid nitrogen is maintained in its liquid state in order to maintain the cryogenic temperature within the container. One or more ultra-violet light sources may be located on the underside of the lid so that the cavity of the container can be irradiated with ultra-violet light while the cavity is being cooled by the Stirling cryocooler. Alternatively (as described further below), the ultra-violet light source(s) may be located on the interior of the container.

By locating one or more ultra-violet light sources on the underside of the lid or on the interior of the container, the container may be sterilised while the Stirling cryocooler is operational. Sterilising the container with UV light causes all components within the container to be decontaminated. That is, sterilising the container causes the solid components of the container itself, the liquid nitrogen used as the working fluid, and any air or other gas within the cavity of the container to be sterilised. This allows the container to be sterilised during transport or storage of a shipping system which comprises a heat engine.

As with the apparatuses described in relation to FIGS. 11 and 12, the cryopreserved sample may be held within a vial or receptacle which is opaque to ultra-violet light, or otherwise shielded from the ultra-violet light from the ultra-violet light source. The method of preparing a shipping system comprising a Stirling cryocooler may be substantially the same as the method 900 described with reference to FIG. 14. However, instead of fitting a bung to the container (as described at step 904 in FIG. 14), the lid comprising the Stirling cryocooler (and optionally the ultra-violet light source(s)) is fitted to the container.

The apparatus 70 shown in FIG. 11 has been described in relation to sterilisation of a container at cryogenic temperatures. However, it will be appreciated that the apparatus 70 shown in FIG. 11 allows for sterilisation of a container which is independent of the temperature of the container. In particular, the apparatus 70 shown in FIG. 11 may allow a container at room temperature to be sterilised.

The cartridge 72 has been described above as having a portion which is arranged to fit within the open end of the container 10. However, other arrangements for irradiating the interior of the container using an ultra-violet light source can also be used. For example, the ultra-violet light source may be located in a lid which sits on top of the container. As a further example, an optical fibre or light guide may be used to transmit the ultra-violet light to the interior of the container, meaning that it may not be necessary to cover the container with a lid in order to irradiate the container interior.

As additional examples, the ultra-violet light source may be located in a separate cartridge which can be placed inside the container. Alternatively, as described above, the ultra-violet light source may be located in a bung such as the bung 20 shown in FIG. 2, the bung 40 shown in FIG. 4, or the bung shown in FIG. 5. As a further alternative, the ultra-violet light source may be located in a lid which comprises a heat engine (as explained above), thereby allowing the container to be sterilised using ultra-violet light while the interior of the container is being cooled to a cryogenic temperature.

Additionally or alternatively, the ultra-violet light sources may be provided on or within the main body of the container (for example, provided on or within the walls and/or on or within the base of the container), or in a component of the container such as the thermal mass 18 of the container 10 shown in FIG. 1. Such an arrangement is shown in FIG. 16, which shows alternative apparatus 100 for sterilising a container for retaining, in particular transporting, cryopreserved samples (i.e. alternative to the apparatus 70 shown in FIG. 11). It should be noted, however, that apparatus for sterilising a container for retaining, in particular transporting, cryopreserved samples according to the methods described herein may comprise the ultra-violet light source 74 of the apparatus 70 shown in FIG. 11 in addition to the ultra-violet light source 104 of the apparatus 100 shown in FIG. 16.

The apparatus 100 shown in FIG. 16 comprises a bung 102 (or other closure) for closing an open end of the container 10. An ultra-violet light source 104 is located on the inside of the wall 12 of the container 10, so that the ultra-violet light source 104 is located within the cavity 16 of the container 10. This means that the ultra-violet light source 104 is arranged to irradiate the interior of the container 10. The ultra-violet light source 104 is powered by a battery 106 located within the walls 12 of the container 10. The ultra-violet light source 104 is controlled using a switch 108 which is positioned on the outside of the wall 12 of the container 10.

Locating the ultra-violet light sources on or within the main body of the container or in a component of the container allows the interior of the container to be sterilised while the interior of the container is being warmed up. Sterilisation of the container during the warming process may also be facilitated by locating the ultra-violet light source in a lid or cartridge which does not comprise insulation so that heat transfer from the ambient environment to the interior of the cavity is permitted through the lid.

If the ultra-violet light sources are located on or within the main body of the container or in a component of the container, the sterilising method 800 described in relation to FIG. 13 may be adapted accordingly. Specifically, the method would not require a bung or other device to be removed from the container in order for a separate cartridge to be fitted. This means that the container interior can be irradiated while an insulating bung (such as the bungs shown in FIG. 2, FIG. 4, and FIG. 5, shown generally as bung 102 in FIG. 16) is fitted to the container, or while a heat engine is attached to the container. Consequently, the method would comprise the steps of activating the ultra-violet light source in the main body of the container or in the component of the container to irradiate the interior of the container with ultra-violet light. The method would then further comprise irradiating the interior of the container with ultra-violet light for a sufficient length of time to ensure that the interior of the container is sterilised. Once the container interior has been sterilised, the ultra-violet light source can be deactivated. Sterilising the container interior in this way means that the container interior may be sterilised in transit (for example, while the container interior is at a cryogenic temperature), while the container is being cooled (for example, from an ambient temperature to a cryogenic temperature), while the container is being warmed (for example, from a cryogenic temperature to an ambient temperature), or while the container is between uses (for example, while the container interior is at an ambient temperature).

Optionally, the apparatus 70 shown in FIG. 11 and/or the apparatus 100 shown in FIG. 16 may include a plurality of ultra-violet light sources. The ultra-violet light sources may be located in the same component of the apparatus (for example, multiple ultra-violet light sources located in the cartridge) or in different components of the apparatus (for example, one or more ultra-violet light sources located in the cartridge and one or more ultra-violet light sources located in the walls of the container).

When a plurality of ultra-violet light sources are used in the apparatus, each ultra-violet light source may emit a different wavelength of ultra-violet light so that a range of ultra-violet wavelengths are used during sterilisation of the container.

When a plurality of ultra-violet light sources are used in the apparatus, a specified portion of the ultra-violet light sources may be activated so that different intensities of ultra-violet light are provided to different parts of the container. For example, the ultra-violet light sources may be controlled so that only part of the interior of the container is irradiated. By irradiating a portion of the container interior, the container interior may be sterilised without irradiating the cryopreserved sample. In some examples, the plurality of ultra-violet light sources may be arranged to provide increased sterilisation of the portion of the base facing the cavity, relative to other surfaces of the container facing the cavity.

Optionally, the ultra-violet light source(s) may comprise an optical fibre or light guide which transmits ultra-violet light to the interior of the container. Transmitting ultra-violet light to the interior of the container using an optical fibre or light guide minimises the heating provided to the interior of the container.

As explained above, the interior of the container may be arranged to reflect ultra-violet light so that the interior of the container is irradiated by reflection of ultra-violet light from the walls and/or the base of the container. Additionally or alternatively, the ultra-violet light source(s) may be arranged within the apparatus so that the whole of the interior of the container is in direct line of sight to one or more of the ultra-violet light source(s).

The ultra-violet light source(s) may be powered by electrical connection to the grid, or by battery operation (as in the apparatus 70, 82 shown in FIGS. 11 and 12 and the apparatus 100 shown in FIG. 16).

Optionally, the ultra-violet light source(s) used in the apparatus may be controlled using an automatic timer. For example, the ultra-violet light source(s) may be controlled so that the interior of the container is irradiated for 10 minutes per day during transport or storage of the cryopreserved sample. Alternatively, the ultra-violet light source(s) may be controlled manually (for example, the apparatus may comprise controls allowing a user to turn off or turn on individual ultra-violet light source(s)). Manual control may be facilitated using a switch (as in the apparatus 70 shown in FIG. 11 and the apparatus 100 shown in FIG. 16). As a further alternative, the ultra-violet light source(s) may be remotely controllable, meaning that the container can be remotely sterilised. To allow remote control of the ultra-violet light source(s), the apparatus may comprise a wireless transceiver which is arranged to receive irradiation control instructions from a remote device.

Optionally, the total irradiation from the ultra-violet light source(s) may be adjusted by adjusting the electrical power supplied to the ultra-violet light source(s) according to requirements. For example, supplying additional electrical power to the ultra-violet light source(s) may increase the intensity of the ultra-violet light provided by each individual ultra-violet light source. Additionally or alternatively, supplying additional electrical power to the ultra-violet light source(s) may cause additional ultra-violet light source(s) to be activated, if only a portion of the ultra-violet light source(s) were activated when a lower amount of electrical power was being supplied.

The adjustment of the total irradiation may be a manual adjustment (for example, the apparatus may comprise a control allowing a user to adjust the total irradiation provided). Alternatively, the adjustment of the total irradiation may be an automatic adjustment (for example, the apparatus may comprise a timer controlling the total irradiation provided by the ultra-violet light source(s) over time).

The total irradiation from the ultra-violet light source(s) may alternatively be adjusted by adjusting the irradiation time of the ultra-violet light source(s) (i.e. the amount of time that each ultra-violet light source is on). The adjustment to the irradiation time of the ultra-violet light source(s) may be a manual adjustment (for example, the apparatus may comprise a control allowing a user to adjust the irradiation time) or an automatic adjustment (for example, using a timer). As a further alternative, the total irradiation provided by the ultra-violet light source(s) may be remotely controllable.

In addition to, or as an alternative to adjusting the irradiation time and/or the electrical power, the wavelength of the ultra-violet light provided by the ultra-violet light source(s) may also be adjustable. Where the ultra-violet light sources are controlled automatically, the function of the ultra-violet light sources may be recoded remotely.

Optionally, the irradiation provided by the ultra-violet light source(s) can be recorded. Recording the irradiation provided by the ultra-violet light sources provides a record of the sterilisation provided by the ultra-violet light source(s), so that an operator can verify whether the container has been sterilised or not.

The irradiation provided by the ultra-violet light source(s) may be recorded by one or more ultra-violet light detectors arranged within the container.

Optionally, the apparatus may comprise an alarm which provides a warning sound when the ultra-violet light source(s) are on. Alternatively or additionally, the apparatus may comprise a warning light which is illuminated when the ultra-violet light source(s) are on.

Optionally, the ultra-violet light source(s) may turn off if the shipping system is opened during an irradiation cycle. To turn off the ultra-violet light source(s) when the shipping system is opened, the connection between the container and the bung or lid may be arranged so that a switch in series with the ultra-violet light source(s) is closed when the lid, bung or cartridge is fitted to the container, where the circuit is then broken when the lid, bung or cartridge is removed from the container. As an alternative, a user may be prevented from opening the shipping system while the ultra-violet light source(s) are on. For example, the bung, lid or cartridge fitted to the container may be locked (e.g. automatically locked) to the container while the ultra-violet light source(s) are activated. That is, the switch that controls the ultra-violet light source(s) may also control a locking mechanism which locks the lid, bung or cartridge to the container while the ultra-violet light source(s) are on.

Optionally, the ultra-violet light source(s) are positioned within the shipping system so that the user is not irradiated with ultra-violet light if the shipping system is opened during an irradiation cycle. For example, a power supply unit may be mounted on the container. The power supply unit is connectable to the mains. The cartridge, when mounted on the container, may form an electrical connection with the power supply unit. This electrical connection may be via pogo pins, via a connection typically found on a kettle, or via any other suitable connector, preferably enabling forming and breaking of the electrical connection to be implemented easily. When the cartridge is removed (i.e. lifted off) from the container, the electrical connection may be broken such that the ultra-violet light turns off automatically. Alternatively, or in addition, a light sensor (using a different frequency of light to the ultra-violet light used for sterilisation) may enable control of the ultra-violet light such that it only operates in environments that are dark.

Optionally, the apparatus comprises a detector arranged to detect whether the lid, bung or cartridge has been removed from the container during the irradiation cycle. If the detector detects that the container has been opened, the detector may send a signal to a controller of the ultra-violet light source(s) so that the ultra-violet light source(s) can be deactivated.

In some examples, the thermal mass may not be included in the container. The bung described herein is suitable for use with such examples.

In some examples, the bung for insulating a container interior from the ambient environment may comprise an insulating segment and one or more barriers for reflecting infrared radiation. In these examples, the insulating segment may be relatively thick. For example, the insulating segment may extend along a majority of a longitudinal extent of the bung.

One or more barriers for reflecting infrared insulation may be disposed adjacent to the insulating segment(s), for example above the insulating segment(s) in use (i.e. further from the cavity of the container than the insulating segment(s)). Since the infrared radiation increases with temperature (proportional to the temperature to the power of four), locating the one or more barriers further from the cavity, i.e. at locations where the temperature is typically higher, may be of most importance for reflection of infrared radiation.

The singular terms "a" and "an" should not be taken to mean "one and only one". Rather, they should be taken to mean "at least one" or "one or more" unless stated otherwise. The word "comprising" and its derivatives including "comprises" and "comprise" include each of the stated features, but does not exclude the inclusion of one or more further features.

The above implementations have been described by way of example only, and the described implementations are to be considered in all respects only as illustrative and not restrictive. It will be appreciated that variations of the described implementations may be made without departing from the scope of the invention. It will also be apparent that there are many variations that have not been described, but that fall within the scope of the appended claims.

## Claims

1. A bung (20, 40, 50) for insulating a container interior (16) from the ambient environment, the bung (20, 40, 50) comprising:
a plurality of insulating segments (26, 54); and
a plurality of barriers (28, 55) for reflecting infrared radiation; wherein at least one of said barriers (28, 55) is arranged between adjacent of said insulating segments (26, 54), and **characterised in that** the bung (20, 40, 50) further comprises a vent channel (36) passing through the plurality of insulating segments and the one or more barriers.

2. The bung (20, 40, 50) according to claim 1, comprising at least three insulating segments (26, 54), and wherein a respective barrier (28, 55) for reflecting infrared radiation is provided between each of said adjacent insulating segments (26, 54).

3. The bung (20, 40, 50) according to claim 1 or claim 2, wherein the bung (20, 40, 50) further comprises a first portion (22, 52) and a second portion (24, 45), wherein the first portion is arranged to be inserted into the container when the bung (20, 40, 50) is fitted to the container (10).

4. The bung (20, 40, 50) according to claim 3, further comprising one or more seals (32, 34) arranged to be compressed between the second portion and the ends, or an interior, of the walls of the container (10) when the bung (20, 40, 50) is fitted to the container (10).

5. The bung (20, 40, 50) according to claim 3 or claim 4, wherein the bung (20, 40, 50) further comprises a clamp (30) arranged to attach the second portion to the walls (12) of the container (10).

6. The bung (20, 40, 50) according to any of claims 1 to 5, wherein the vent channel (36) comprises one or more valves and a cavity in the path of the vent channel, the cavity being located at the region of the vent channel where the temperature of the air in the vent channel is configured to be zero degrees Celsius in use.

7. The bung (20, 40, 50) according to any of claims 1 to 6, wherein the bung (20, 40, 50) further comprises one or more chambers (52), each of the chambers (52) housing at least one of the plurality of insulating segments and at least one of the one or more barriers for reflecting infrared radiation.

8. The bung (20, 40, 50) according to claim 7, wherein each of the one or more of chambers (52) includes a bottom and a side wall, creating a cavity, and wherein at least two of the plurality of insulating segments and at least one of the one or more barriers for reflecting infrared radiation are located within the cavity.

9. The bung (20, 40, 50) according to claim 8, wherein the bung (20, 40, 50) further comprises at least one spacer (55), and wherein each spacer (55) is located between adjacent insulating segments in each of the cavities, and wherein the one or more barriers for reflecting infrared radiation are located on a top and/or bottom surface of at least one of the plurality of insulting segments.

10. The bung (20, 40, 50) according to any of claims 1 to 9, wherein the bung (20, 40, 50) further comprises an ultra-violet light source (74) of a power sufficient to provide sterilisation inside the container (10) in use.

11. A shipping system (38) for retaining cryopreserved samples, the shipping system (38) comprising:
a container (10) comprising a thermal mass (18); and
a bung (20, 40, 50) as claimed in any one of claims 1 to 10.

12. The shipping system (38) of claim 11, wherein:
i) the container (10) comprises at least one sensor and at least one controller;
ii) the bung (20, 40, 50) comprises at least one sensor and at least one controller;
iii) the bung (20, 40, 50) comprises a connector, and wherein the container is in electrical communication with the bung (20, 40, 50) via connection to the connector; and/or
iv) at least one of the bung (20, 40, 50) and the container (10) comprises a transceiver configured to wirelessly transmit information coming from at least one sensor.

13. A method (900) of preparing a shipping system (38) for retaining a cryopreserved sample, the method comprising:
loading (902) a cryopreserved sample into a container (10) such as a vacuum flask; and **characterised by**:
fitting (904) a bung (20, 40, 50) as claimed in any one of claims 1 to 10 to the container (10) to insulate the container interior (16) from the ambient environment.

14. The method (900) according to claim 13, further comprising:
i) cooling the container interior (16) to a desired temperature;
ii) cooling the container interior to the desired temperature by: a) fitting a heat engine to the container (10) to remove heat from the interior of the container (10) or b) by pouring a cryogenic fluid such as liquid nitrogen into the container (10) and subsequently emptying the cryogenic fluid from the container (10); and/or
iii) cooling a thermal mass, such as a metal or metallic block, within the container (10) to a desired temperature.

## Patentansprüche

1. Stopfen (20, 40, 50) zum Isolieren eines Behälterinneren (16) von der Außenumgebung, wobei der Stopfen (20, 40, 50) umfasst:
eine Vielzahl von Isoliersegmenten (26, 54); und
eine Vielzahl von Barrieren (28, 55) zum Reflektieren von Infrarotstrahlung; wobei mindestens eine der Barrieren (28, 55) zwischen benachbarten der Isoliersegmente (26, 54) angeordnet ist, und **dadurch gekennzeichnet, dass** der Stopfen (20, 40, 50) weiter einen Belüftungskanal (36) umfasst, der durch die Vielzahl von Isoliersegmenten und die eine oder die mehreren Barrieren hindurch verläuft.

2. Stopfen (20, 40, 50) nach Anspruch 1, der mindestens drei Isoliersegmente (26, 54) umfasst, und wobei zwischen jedem der benachbarten Isoliersegmente (26, 54) eine entsprechende Barriere (28, 55) zum Reflektieren von Infrarotstrahlung bereitgestellt ist.

3. Stopfen (20, 40, 50) nach Anspruch 1 oder Anspruch 2, wobei der Stopfen (20, 40, 50) weiter einen ersten Abschnitt (22, 52) und einen zweiten Abschnitt (24, 45) umfasst, wobei der erste Abschnitt so angeordnet ist, dass er in den Behälter eingeführt wird, wenn der Stopfen (20, 40, 50) an dem Behälter (10) angebracht wird.

4. Stopfen (20, 40, 50) nach Anspruch 3, weiter umfassend eine oder mehrere Dichtungen (32, 34), die so angeordnet sind, dass sie zwischen dem zweiten Abschnitt und den Enden oder einem Inneren der Wände des Behälters (10) zusammengedrückt werden, wenn der Stopfen (20, 40, 50) an dem Behälter (10) angebracht wird.

5. Stopfen (20, 40, 50) nach Anspruch 3 oder Anspruch 4, wobei der Stopfen (20, 40, 50) weiter eine Klemme (30) umfasst, die so angeordnet ist, dass sie den zweiten Abschnitt an den Wänden (12) des Behälters (10) befestigt.

6. Stopfen (20, 40, 50) nach einem der Ansprüche 1 bis 5, wobei der Belüftungskanal (36) ein oder mehrere Ventile und einen Hohlraum im Verlauf des Belüftungskanals umfasst, wobei sich der Hohlraum in dem Bereich des Belüftungskanals befindet, in dem die Temperatur der Luft in dem Belüftungskanal so konfiguriert ist, dass sie beim Gebrauch null Grad Celsius ist.

7. Stopfen (20, 40, 50) nach einem der Ansprüche 1 bis 6, wobei der Stopfen (20, 40, 50) weiter eine oder mehrere Kammern (52) umfasst, wobei jede der Kammern (52) mindestens eines der Vielzahl von Isoliersegmenten und mindestens eine der einen oder der mehreren Barrieren zum Reflektieren von Infrarotstrahlung beherbergt.

8. Stopfen (20, 40, 50) nach Anspruch 7, wobei jede der einen oder der mehreren Kammern (52) einen Boden und eine Seitenwand einschließt, wodurch ein Hohlraum entsteht, und wobei sich mindestens zwei der Vielzahl von Isoliersegmenten und mindestens eine der einen oder mehreren Barrieren zum Reflektieren von Infrarotstrahlung innerhalb des Hohlraums befinden.

9. Stopfen (20, 40, 50) nach Anspruch 8, wobei der Stopfen (20, 40, 50) weiter mindestens einen Abstandshalter (55) umfasst, und wobei sich jeder Abstandshalter (55) zwischen benachbarten Isoliersegmenten in jedem der Hohlräume befindet, und wobei sich die eine oder die mehreren Barrieren zum Reflektieren von Infrarotstrahlung auf einer Ober- und/oder Unterseite von mindestens einem der Vielzahl von Isoliersegmenten befinden.

10. Stopfen (20, 40, 50) nach einem der Ansprüche 1 bis 9, wobei der Stopfen (20, 40, 50) weiter eine Ultraviolettlichtquelle (74) mit einer Leistung umfasst, die ausreicht, um beim Gebrauch eine Sterilisation innerhalb des Behälters (10) bereitzustellen.

11. Transportsystem (38) zum Aufbewahren kryokonservierter Proben, wobei das Transportsystem (38) umfasst:
einen Behälter (10), der eine thermische Masse (18) umfasst; und
einen Stopfen (20, 40, 50) nach einem der Ansprüche 1 bis 10.

12. Transportsystem (38) nach Anspruch 11, wobei:
i) der Behälter (10) mindestens einen Sensor und mindestens eine Steuereinheit umfasst;
ii) der Stopfen (20, 40, 50) mindestens einen Sensor und mindestens eine Steuereinheit umfasst;
iii) der Stopfen (20, 40, 50) einen Stecker umfasst und wobei der Behälter über eine Verbindung mit dem Stecker in elektrischer Verbindung mit dem Stopfen (20, 40, 50) steht; und/oder
iv) mindestens einer von dem Stopfen (20, 40, 50) und dem Behälter (10) einen Transceiver umfasst, der so konfiguriert ist, dass er Informationen drahtlos überträgt, die von mindestens einem Sensor kommen.

13. Verfahren (900) zum Vorbereiten eines Transportsystems (38) zum Aufbewahren einer kryokonservierten Probe, wobei das Verfahren umfasst:
Laden (902) einer kryokonservierten Probe in einen Behälter (10), wie zum Beispiel eine Vakuumflasche; und **gekennzeichnet durch**:
Anbringen (904) eines Stopfens (20, 40, 50) nach einem der Ansprüche 1 bis 10 an dem Behälter (10), um das Behälterinnere (16) von der Außenumgebung zu isolieren.

14. Verfahren (900) nach Anspruch 13, weiter umfassend:
i) Kühlen des Behälterinneren (16) auf eine gewünschte Temperatur;
ii) Kühlen des Behälterinneren auf die gewünschte Temperatur durch: a) Anbringen einer Wärmekraftmaschine an dem Behälter (10), um Wärme aus dem Inneren des Behälters (10) zu entfernen, oder b) Einfüllen eines kryogenen Fluids, wie zum Beispiel flüssigem Stickstoff, in den Behälter (10) und anschließendes Entleeren des kryogenen Fluids aus dem Behälter (10); und/oder
iii) Kühlen einer thermischen Masse, wie zum Beispiel eines Metalls oder Metallblocks, innerhalb des Behälters (10) auf eine gewünschte Temperatur.

## Revendications

1. Bouchon (20, 40, 50) pour isoler un intérieur (16) de récipient de l'environnement ambiant, le bouchon (20, 40, 50) comprenant :
une pluralité de segments isolants (26, 54) ; et
une pluralité de barrières (28, 55) pour réfléchir un rayonnement infrarouge ; dans lequel au moins l'une desdites barrières (28, 55) est agencée entre des segments adjacents parmi lesdits segments isolants (26, 54), et **caractérisé en ce que** le bouchon (20, 40, 50) comprend en outre un canal d'évent (36) traversant la pluralité de segments isolants et les une ou plusieurs barrières.

2. Bouchon (20, 40, 50) selon la revendication 1, comprenant au moins trois segments isolants (26, 54), et dans lequel une barrière (28, 55) respective pour réfléchir le rayonnement infrarouge est prévue entre chacun desdits segments isolants (26, 54) adjacents.

3. Bouchon (20, 40, 50) selon la revendication 1 ou la revendication 2, dans lequel le bouchon (20, 40, 50) comprend en outre une première partie (22, 52) et une seconde partie (24, 45), dans lequel la première partie est agencée pour être insérée dans le récipient lorsque le bouchon (20, 40, 50) est monté sur le récipient (10).

4. Bouchon (20, 40, 50) selon la revendication 3, comprenant en outre un ou plusieurs joints (32, 34) agencés pour être comprimés entre la seconde partie et les extrémités, ou un intérieur, des parois du récipient (10) lorsque le bouchon (20, 40, 50) est monté sur le récipient (10).

5. Bouchon (20, 40, 50) selon la revendication 3 ou la revendication 4, dans lequel le bouchon (20, 40, 50) comprend en outre une pince (30) agencée pour fixer la seconde partie aux parois (12) du récipient (10).

6. Bouchon (20, 40, 50) selon l'une quelconque des revendications 1 à 5, dans lequel le canal d'évent (36) comprend une ou plusieurs vannes et une cavité dans le trajet du canal d'évent, la cavité étant située au niveau de la région du canal d'évent où la température de l'air dans le canal d'évent est configurée pour être de zéro degré Celsius lors de l'utilisation.

7. Bouchon (20, 40, 50) selon l'une quelconque des revendications 1 à 6, dans lequel le bouchon (20, 40, 50) comprend en outre une ou plusieurs chambres (52), chacune des chambres (52) hébergeant au moins l'un de la pluralité de segments isolants et au moins l'une des une ou plusieurs barrières pour réfléchir le rayonnement infrarouge.

8. Bouchon (20, 40, 50) selon la revendication 7, dans lequel chacune des une ou plusieurs chambres (52) inclut un fond et une paroi latérale, créant une cavité, et dans lequel au moins deux de la pluralité de segments isolants et au moins une des une ou plusieurs barrières pour réfléchir le rayonnement infrarouge sont situés à l'intérieur de la cavité.

9. Bouchon (20, 40, 50) selon la revendication 8, dans lequel le bouchon (20, 40, 50) comprend en outre au moins une entretoise (55), et dans lequel chaque entretoise (55) est située entre des segments isolants adjacents dans chacune des cavités, et dans lequel les une ou plusieurs barrières pour réfléchir le rayonnement infrarouge sont situées sur une surface supérieure et/ou inférieure d'au moins l'un de la pluralité de segments isolants.

10. Bouchon (20, 40, 50) selon l'une quelconque des revendications 1 à 9, dans lequel le bouchon (20, 40, 50) comprend en outre une source de lumière ultraviolette (74) d'une puissance suffisante pour assurer la stérilisation à l'intérieur du récipient (10) en cours d'utilisation.

11. Système d'expédition (38) pour conserver des échantillons cryoconservés, le système d'expédition (38) comprenant :
un récipient (10) comprenant une masse thermique (18) ; et
un bouchon (20, 40, 50) selon l'une quelconque des revendications 1 à 10.

12. Système d'expédition (38) selon la revendication 11, dans lequel :
i) le récipient (10) comprend au moins un capteur et au moins un dispositif de commande ;
ii) le bouchon (20, 40, 50) comprend au moins un capteur et au moins un dispositif de commande ;
iii) le bouchon (20, 40, 50) comprend un connecteur, et dans lequel le récipient est en communication électrique avec le bouchon (20, 40, 50) via une connexion au connecteur ; et/ou
iv) au moins un du bouchon (20, 40, 50) et du récipient (10) comprend un émetteur-récepteur configuré pour transmettre sans fil des informations provenant d'au moins un capteur.

13. Procédé (900) de préparation d'un système d'expédition (38) pour la conservation d'un échantillon cryoconservé, le procédé comprenant :
le chargement (902) d'un échantillon cryoconservé dans un récipient (10) tel qu'une fiole sous vide ; et **caractérisé par** :
le montage (904) d'un bouchon (20, 40, 50) selon l'une quelconque des revendications 1 à 10 sur le récipient (10) pour isoler l'intérieur (16) du récipient de l'environnement ambiant.

14. Procédé (900) selon la revendication 13, comprenant en outre :
i) le refroidissement de l'intérieur (16) du récipient à une température souhaitée ;
ii) le refroidissement de l'intérieur du récipient à la température souhaitée par : a) montage d'un moteur thermique sur le récipient (10) pour éliminer la chaleur de l'intérieur du récipient (10) ou b) par versement d'un fluide cryogénique tel que de l'azote liquide dans le récipient (10) et ensuite vidage du fluide cryogénique du récipient (10) ; et/ou
iii) refroidissement d'une masse thermique, telle qu'un bloc en métal ou métallique, à l'intérieur du récipient (10) à une température souhaitée.
